# EUROPEAN PATENT SPECIFICATION

(11) **EP 2 531 261 B1**
(45) Date of publication and mention of the grant of the patent: **31.08.2016**
(21) Application number: 11737851.3
(22) Date of filing: 01.02.2011
(51) Int. Cl.: A61P 3/10, A61K 39/395, A61K 31/7088

(54) **METHODS FOR DIAGNOSIS AND TREATMENT OF NON-INSULIN DEPENDENT DIABETES MELLITUS**
VERFAHREN ZUR DIAGNOSE UND BEHANDLUNG VON NICHT-INSULINABHÄNGIGER DIABETES MELLITUS
MÉTHODES DIAGNOSTIQUES ET TRAITEMENT DU DIABÈTE NON INSULINODÉPENDANT

(30) Priority: 01.02.2010 US 300400 P
(43) Date of publication of application: 12.12.2012
(73) Proprietor: The Board of Trustees of the Leland Stanford Junior University, Stanford CA 94305-2038 (US)
(72) Inventor: BUTTE, Atul J., Stanford, California 94305 (US); KODAMA, Keiichi, Stanford, California 94305 (US)
(74) Representative: Brasnett, Adrian Hugh
(86) International application number: PCT/US2011/023299
(87) International publication number: WO 2011/094731

(56) References cited:
- EP-A1- 1 647 556
- US-A- 6 001 356
- US-A- 6 150 162
- US-B1- 6 653 285
- MARUTSUKA K ET AL: "Pathogenetic implications of hyaluronan-induced modification of vascular smooth muscle cell fibrinolysis in diabetes.", CORONARY ARTERY DISEASE 1998, vol. 9, no. 4, 1998, pages 177-184, XP8163425, ISSN: 0954-6928
- YEVDOKIMOVA NATALIA Y ET AL: "Hyaluronic acid production and CD44 expression in cultured dermal fibroblasts of patients with non-insulin-dependent diabetes mellitus with and without chronic ulcers on the lower extremity.", WOUND REPAIR AND REGENERATION : OFFICIAL PUBLICATION OF THE WOUND HEALING SOCIETY [AND] THE EUROPEAN TISSUE REPAIR SOCIETY 2005 MAR-APR, vol. 13, no. 2, March 2005 (2005-03), pages 181-188, XP002700092, ISSN: 1067-1927
- LUCARINI GUENDALINA ET AL: "Involvement of vascular endothelial growth factor, CD44 and CD133 in periodontal disease and diabetes: an immunohistochemical study.", JOURNAL OF CLINICAL PERIODONTOLOGY JAN 2009, vol. 36, no. 1, January 2009 (2009-01), pages 3-10, XP002700093, ISSN: 1600-051X
- DATABASE MEDLINE [Online] US NATIONAL LIBRARY OF MEDICINE (NLM), BETHESDA, MD, US; September 2008 (2008-09), IEVDOKIMOVA N IU: "[Hyaluronic acid, receptor CD44, and their role in diabetic complications].", XP002700094, Database accession no. NLM19248616
- DATABASE MEDLINE [Online] US NATIONAL LIBRARY OF MEDICINE (NLM), BETHESDA, MD, US; March 2003 (2003-03), LIU LIMEI ET AL: "The heparan sulfate proteoglycan gene polymorphism: association with type 2 diabetic nephropathy in Chinese.", XP002700095, Database accession no. NLM12708751
- MÜLLER GÜNTER: "Personalized prognosis and diagnosis of type 2 diabetes--vision or fiction?", PHARMACOLOGY 2010, vol. 85, no. 3, 2010, pages 168-187, XP002700096, ISSN: 1423-0313
- TALMUD PHILIPPA J ET AL: "Utility of genetic and non-genetic risk factors in prediction of type 2 diabetes: Whitehall II prospective cohort study.", BMJ (CLINICAL RESEARCH ED.) 2010, vol. 340, 14 January 2010 (2010-01-14), page b4838, XP002700097, ISSN: 1756-1833
- TABARA YASUHARU ET AL: "Replication study of candidate genes associated with type 2 diabetes based on genome-wide screening.", DIABETES FEB 2009, vol. 58, no. 2, February 2009 (2009-02), pages 493-498, XP002700098, ISSN: 1939-327X
- KIEFER ET AL.: 'Neutralization of osteopontin inhibits obesity-induced inflammation and insulin resistance.' DIABETES vol. 59, no. 4, April 2010, pages 935 - 946
- BERTOLA ET AL.: 'Elevated expression of osteopontin may be related to adipose tissue macrophage accumulation and liver steatosis in morbid obesity.' DIABETES vol. 58, no. 1, January 2009, pages 125 - 133

## Description

### STATEMENT OF GOVERNMENTAL SUPPORT

This invention was made with U.S. Government support under National Institutes of Health Grant LM008261. The Government has certain rights in this invention.

### BACKGROUND OF THE INVENTION

Diabetes is a metabolic disease that occurs when the pancreas does not produce enough of the hormone insulin to regulate blood sugar ("type 1 diabetes mellitus") or, alternatively, when the body cannot effectively use the insulin it produces ("type 2 diabetes mellitus").

According to recent estimates by the World Health Organization, more than 200 million people worldwide have diabetes, whereby 90% suffer from type 2 diabetes mellitus. Typical long term complications include development of neuropathy, retinopathy, nephropathy, generalized degenerative changes in large and small blood vessels and increased susceptibility to infection. Since individuals with type 2 diabetes still have a residual amount of insulin available in contrast to type 1 diabetic individuals, who completely lack the production of insulin, type 2 diabetes only surfaces gradually and is often diagnosed several years after onset, once complications have already arisen.

Insulin resistance occurs in 25% of non-diabetic, non-obese, apparently healthy individuals, and predisposes them to both diabetes and coronary artery disease. Hyperglycemia in type II diabetes is the result of both resistance to insulin in muscle and other key insulin target tissues, and decreased beta cell insulin secretion. Longitudinal studies of individuals with a strong family history of diabetes indicate that the insulin resistance precedes the secretory abnormalities. Prior to developing diabetes these individuals compensate for their insulin resistance by secreting extra insulin. Diabetes results when the compensatory hyperinsulinemia fails. The secretory deficiency of pancreatic beta cells then plays a major role in the severity of the diabetes.

Reaven (1988) Diabetes 37:1595-607 were the first to have investigated insulin resistant, non-diabetic, healthy individuals from the general population who are non-obese. Strikingly, they observed that 25% of them have insulin resistance that is of a similar magnitude to that seen in type II diabetes patients. These individuals compensate by having insulin levels that are 3-4 times higher than normal. These elevated insulin levels are sufficient to maintain normoglycemia. Others have also confirmed that a large proportion of the non-diabetic population is insulin resistant. These insulin resistant, non-diabetic individuals have a much higher risk for developing type II diabetes than insulin sensitive subjects.

However, even without developing hyperglycemia and diabetes, these insulin resistant individuals pay a significant price in terms of general health. Insulin resistance results in an increased risk for having elevated plasma triglycerides (TG), lower high density lipoproteins (HDL), and high blood pressure, a cluster of abnormalities that have been termed by different investigators as either Syndrome X, the insulin resistance syndrome, or the metabolic syndrome. It is believed that either the hyperinsulinemia, insulin resistance, or both play a direct role in causing these abnormalities. Data from ethnic, family, and longitudinal studies suggest that a major component of resistance is inherited.

Marutsuka et al, Coronary Artery Disease 1998, vol. 9, no. 4, 1998, pages 177-184 describe pathogenetic implications of hyaluronan-induced modification of vascular smooth muscle cell fibrinolysis in diabetes. EP1647556 relates to peptidic compounds and derivatives thereof for the treatment of human diseases through inhibition of signaling via growth factors.

Type 2 diabetes mellitus is a multigenic disease; its already high prevalence in adults worldwide and its increasing prevalence in both adults and children indicate an urgent need to assess the genetic susceptibility to this disease as early in life as possible and to identify therapeutic interventions to prevent the onset of type 2 diabetes mellitus and to treat the disease.

### SUMMARY OF THE INVENTION

The present disclosure relates to methods and compositions for the treatment and diagnosis of diseases related to hyperglycemic conditions, including diabetes, insulin resistance, and the like. Genetic polymorphisms are shown to be associated with disease susceptibility, and their detection is used in the diagnosis of a predisposition to these conditions. Polymorphisms of interest include single nucleotide polymorphisms, splice variants, deletions, insertions, and the like. As shown herein, polymorphisms in the CD44 locus and the CD44 ligand (SPP1, osteopontin), as well as the HDC locus, are predictive of a susceptibility in an individual to development of type 2 diabetes. Serum levels of CD44 are also diagnostic of insulin resistance. Methods of treatment target the product of these genes, particularly targeting CD44 and/or osteopontin.

The present invention provides an inhibitor of CD44, which inhibitor is an antibody, for use in a method of treating insulin resistance, the method comprising administering the inhibitor of CD44 to an individual having type 2 diabetes, or diagnosed as susceptible to type 2 diabetes. The invention also provides a kit for assessing susceptibility to type 2 diabetes mellitus in an individual, the kit comprising reagents for selectively determining the presence or absence of at least one polymorphic allele in a biological sample from said individual, wherein the polymorphic allele is selected from the polymorphisms set forth in SEQ ID NO:1-4, and wherein the presence of the at least one SNP indicates susceptibility to type 2 diabetes. The invention is defined by the accompanying claims.

Described herein are methods for prevention or treatment of type 2 diabetes, by administering to an individual an inhibitor of CD44 activity. The CD44 inhibitor is optionally selective for CD44 splice variants expressed in cells related to the development of type 2 diabetes, e.g. adipose tissue macrophages, *etc.* Inhibitors described herein may be targeted to a cell of interest, e.g. adipose tissue macrophages. Inhibitors described herein include, without limitation, inhibitors of the interaction between the CD44 receptor and osteopontin, and inhibitors of the interaction between CD44 and hyaluronic acid. Such inhibitors include, without limitation, antibodies, nucleic acid inhibitors such as antisense oligonucleotides, RNAi molecules, *etc;* inhibitory osteopontin peptides, small molecules inhibitors, and the like. In some embodiments, the susceptibility of the individual to developing disease is assessed prior to initiation of treatment, for example using the predictive methods of the invention.

Also described herein are methods for determining the susceptibility of an individual to type 2 diabetes mellitus (T2D), by determining the presence of disease-associated polymorphisms in the genetic sequences encoding one or more of CD44, SPP1 and HDC. In certain embodiments, multiple loci are analyzed. Polymorphisms within CD44, SPP1 and HDC are described and applied alone or in combination with other T2D variants to predict individual susceptibility to type 2 diabetes mellitus. In some embodiments the polymorphism is an SNP. In other embodiments the polymorphism is a splice variant.

In other embodiments of the invention, methods are provided for identifying compounds useful in the prevention or treatment of T2D, for example small molecule inhibitors and other drug candidates. In such methods, the effect of a candidate agent on inhibiting activity of CD44 is determined. The compound is optionally then further tested in a model of T2D, which model may be a cell culture model, an animal model, or the like. Included in such models are isolated adipose tissue macrophages, which express CD44 and which have been shown to have an anti-adipogenic effect in culture models.

In other embodiments, kits are provided for assessing susceptibility to Type 2 diabetes in a human individual, the kit comprising reagents for selectively determining the presence or absence of one or more disease associated polymorphisms as defined herein.

The above summary is not intended to include all features and aspects of the present invention nor does it imply that the invention must include all features and aspects discussed in this summary.

### BRIEF DESCRIPTION OF THE FIGURES

The accompanying drawings illustrate embodiments of the invention and, together with the description, serve to explain the invention. These drawings are offered by way of illustration and not by way of limitation; it is emphasized that the various features of the drawings are not to-scale.

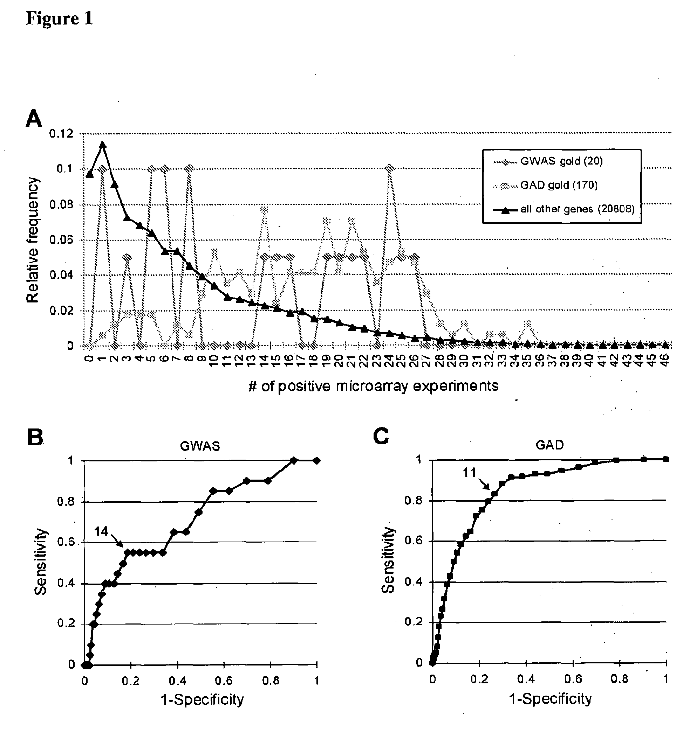

Figure 1 Validation of T2D microarray experiments database. (A) Known T2D-associated genes (diamonds (◆), 20 genes previously detected by GWAS; squares (■), 170 genes extracted from the NIH Genetic Association Database, GAD) are represented by the number of times they are significantly differentially expressed in a set of 69 microarray studies, against all other genes in the genome (triangles (A)). The known T2D-associated gene implicated in the most experiments was leptin receptor (*LEPR*), positive in 35 of 69 microarray experiments (Table 3). The maximum number of positive microarray experiments for any gene was 46. (B and C) ROC curves were drawn by plotting sensitivity and specificity in the rediscovery of the 20 GWAS (B) and 170 GAD (C) genes using all 69 microarray data. The best sensitivity and specificity are 55% and 81%, respectively, in rediscovery of the 20 GWAS genes by the threshold of ≥ 14 positive microarray experiments (B), and 83% and 73% in rediscovery of the 170 GAD genes by the threshold of ≥ 11 positive microarray experiments (C).

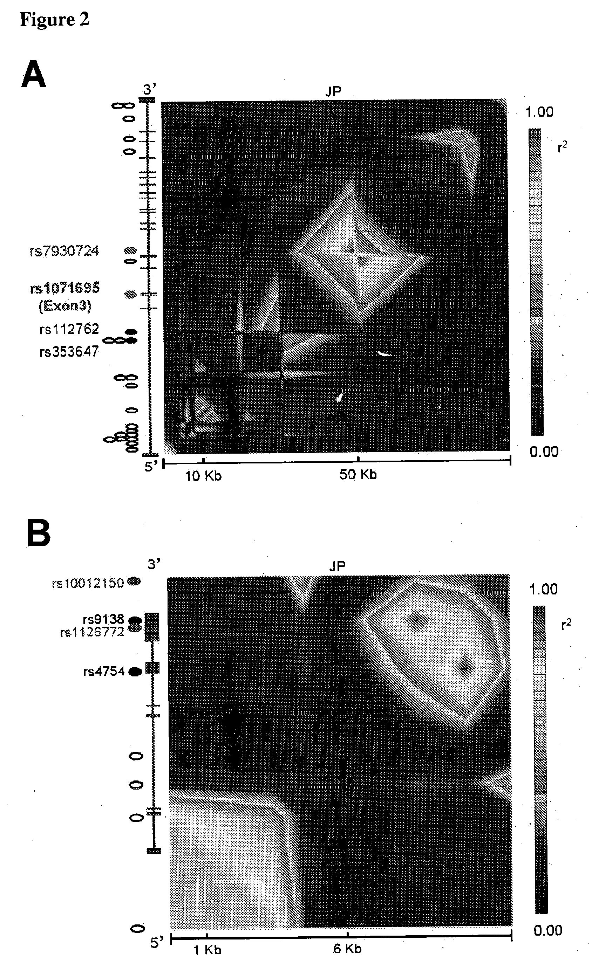

Figure 2 Significant SNPs in Linkage Disequilibrium map. The SNPs genotyped in our targeted association studies (stage I, and II) are indicated with open/filled circles in the LD map of *CD44* and *OPN* (black filled circle; *P* < 0.05, red filled circle; *P* < Bonferroni threshold). The LD structure was plotted using GOLD software based on SNP data in 192 random Japanese (JP) control subjects in stage I. The axes are scaled by distance markers (kb). The exons and introns are shown on the vertical axis. (A) *CD44;* Three tag SNPs (rs353647, rs112762 and rs7930724) were selected from 23 screened tag SNPs of *CD44* in the initial study (P < 0.05; stage I) (Table 5), and further genotyped in the second cohort (stage II). When the studies were meta-analyzed, SNPs rs353647 and rs112762 were nominally, and rs7930724 was significantly associated with T2D (rs7930724; OR = 1.23, P = 2.0 × 10⁻³) (Table 1). The coding SNP rs1071695 (exon 3) was identified by direct-sequencing the moderate LD blocks (exon 2-13) including these 3 tag SNPs, and its strongly significant association with T2D was confirmed using the same cohorts (rs1071695; OR = 1.43, *P* = 3.9 × 10⁻⁴). (see Table 6 for haplotype analysis) (B) *OPN;* Three tag SNPs (rs4754, rs9138 (3'UTR) and rs10012150 (3'UTR)) were selected from 10 screened tag SNPs of *OPN* (*P* < 0.05; stage I) (Supplemental Table 7). The 3 tag SNPs were further tested (stage II). SNP rs9138 was nominally, and rs10012150 was significantly associated with T2D when meta-analyzed (rs10012150; OR = 1.15, P = 1.5 × 10⁻³). Another 3'UTR SNP (rs1126772) was identified in the LD block of these 3 tag SNPs, and its significant association with T2D was also confirmed (rs1126772; OR = 1.14, P = 4.4 × 10⁻³) (Table 1).

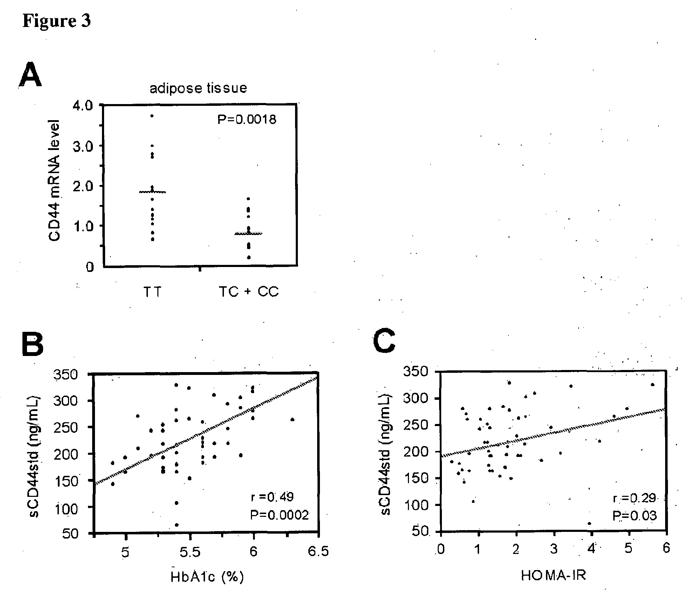

Figure 3: CD44 functional experiments in human subjects. (A) The *CD44* mRNA expression was compared between age, gender, BMI-matched subjects with two risk alleles (TT, n=13) and one (TC, n=12) or no (CC, n=1) risk allele of rs1071695. Subcutaneous fat removal was performed for cosmetic purposes. All the patients were non-diabetic subjects (age (yr) 59.5 ± 15.3, gender (M/F) 2/24, BMI (kg/m²) 22.9 ± 2.3, HbA1c (%) 5.1 ± 0.57). (B and C) The correlation between serum levels of standard soluble CD44 (sCD44std) and indices of insulin resistance, HbA1c (B) and HOMA-IR (C), was determined by using linear regression model estimated with minimal square method in non-diabetic subjects (n=55: age (yr); 60.3 ± 15, gender (M/F); 36/19, BMI (kg/m²); 23.2 ± 4.3)

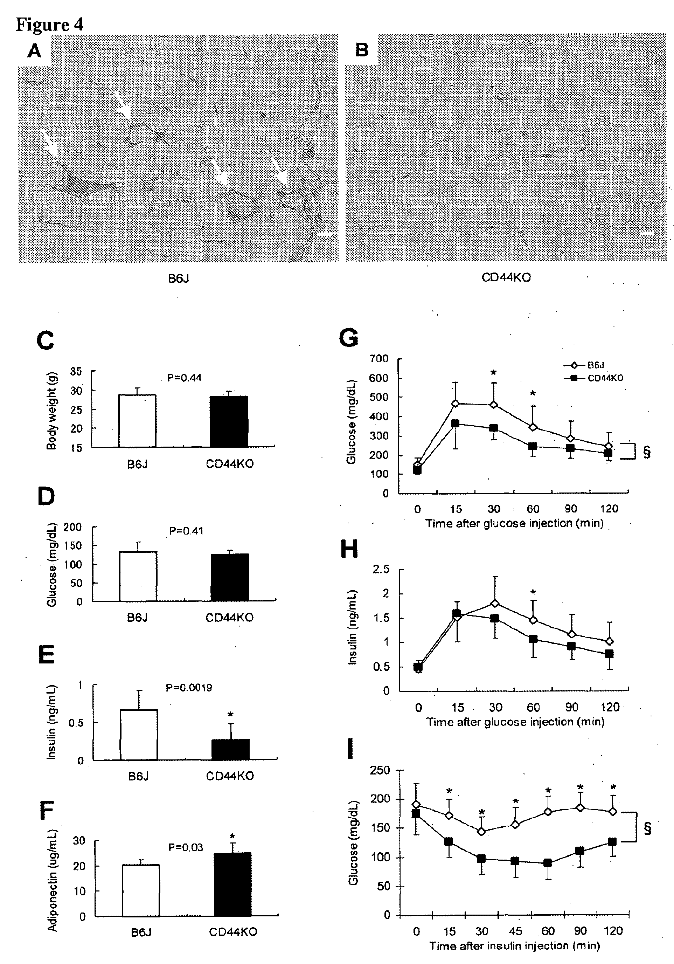

Figure 4: CD44 functional experiments in mouse models. (A and B) Visceral (epididymal) adipose tissues isolated from diabetes-prone WT (A) and CD44-KO (B) mice were stained with anti Mac-2 (macrophage marker) antibody followed by DAB (brown), and counterstained with hematoxylin (blue). Arrows show macrophage clusters surrounding adipocytes. Bar = 20 µm. (C-I) Improvement of glucose homeostasis in *CD44-KO* mice. Body weights (C) were measured prior to experiments. Blood glucose (D), serum insulin (E) and serum adiponectin (F) were measured in *CD44-KO* mice (n=7) and age-matched WT (n=16; diabetes-prone) after a 14-hour overnight fast. **P* < 0.05, versus WT. (G and H) Glucose tolerance tests (i.p. with glucose (2 g/kg body weight)) were performed in *CD44-*KO mice (n=7) and age-matched WT (n=15) after a 14-hour overnight fast. Blood glucose (G) and serum insulin (H) were measured at the indicated time points. (I) Insulin tolerance tests (i.p. with insulin (1.0 unit/kg body weight)) were performed in *CD44-KO* mice (n=7) and age-matched WT (n=16) after a 4-hour fast. All experiments on mice were conducted between 14 and 20 weeks of age. **P* < 0.05, versus WT at each time point. §P < 0.05, versus WT (AUC values). All data are presented as mean ± SD.

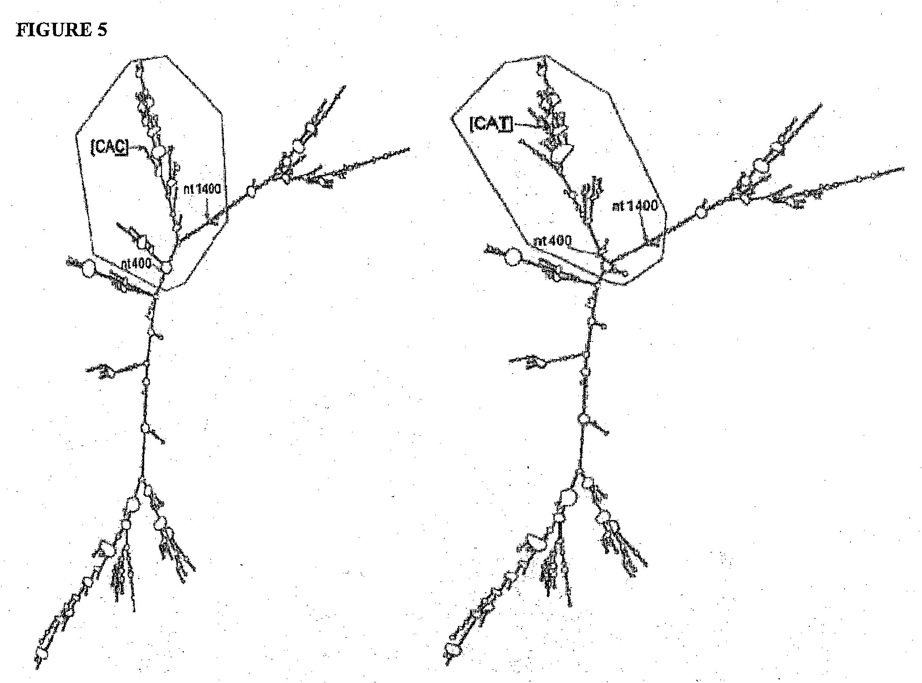

Figure 5: Cumulative effects of *CD44* and *OPN* risk alleles on T2D in the Japanese population. The proportions of participants with increasing numbers of risk alleles of *CD44* and *OPN* are indicated by color bars (red: controls, blue: T2D cases). The odds ratios for T2D for participants carrying increasing numbers of risk alleles are presented by yellow triangles, in comparison to those that have no or only one risk allele. The participants with four risk alleles had an OR of 3.21 [1.39-7.39] (overall *P* = 7.5 × 10⁻³). Each additional risk allele increased odds of disease by 1.21 [1.07-1.37] (*P* = 2.1 × 10⁻³) times.

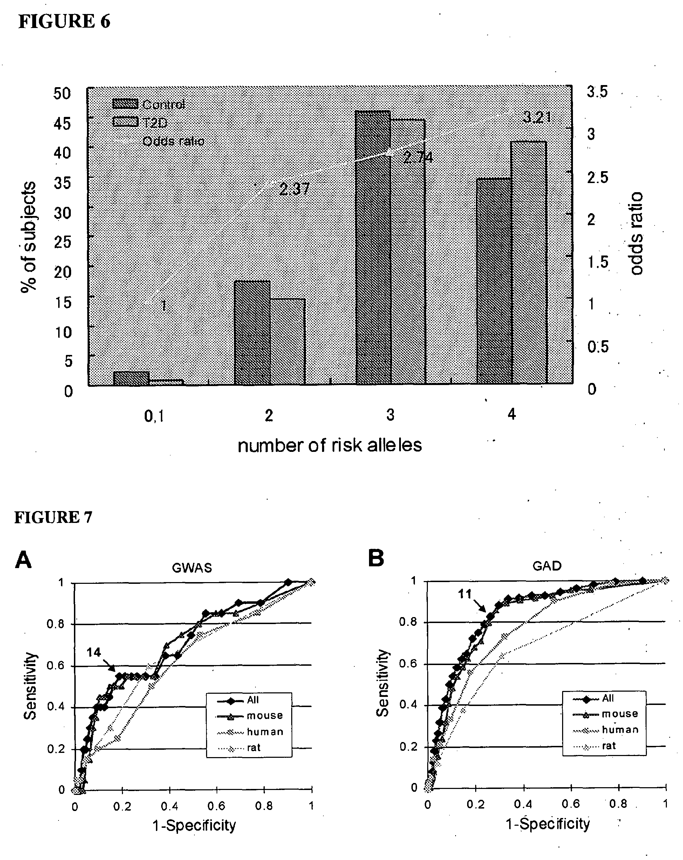

Figure 6: Optimal secondary structures of full-length *CD44* mRNA transcripts. *CD44* mRNA full-length sequence (NM_001001391; 5'UTR-coding sequence-3'UTR) with and without the associated synonymous SNP (rs1071695; His [CAC]/[CAT]: nt 689) was used for optimal secondary folding structure building using Mfold. An obvious change of folding pattern was observed in the protein coding region (nt 435-1517) between the [CAC] and [CAT] *CD44* mRNA.
Figure 7: ROC curves for all 69 combined microarray data and each set of mouse, human, and rat microarray experiments **(A** and **B)** ROC curves were drawn by plotting sensitivity and specificity in the rediscovery of the 20 GWAS **(A)** and 170 GAD **(B)** gold-standard genes using all 69 (human, mouse, and rat) microarray data (black line), just the 48 mouse experiments (red line), just the 13 human experiments (blue line), and just the 8 rat experiments (green line). The best sensitivity and specificity are achieved in all 69 microarray data (black). The set of mouse microarrays (red) showed better performance than human (blue) and rat microarrays (green). These findings demonstrate that more microarray experiments yield better performance in recalling gold-standard genes, independently of species.
Figure 8: Significant SNPs in Linkage Disequilibrium map of *CD44 and OPN.* The SNPs genotyped in our targeted association study and WTCCC GWAS are indicated with open/filled circles in the LD maps of *CD44* and *OPN* (JP and CEU). The graphic pictures of LD structure of gene region were drawn by plotting pairwise *r²* values of SNPs in our stage I Japanese (JP) controls or HapMap European-ancestry (CEU) population using GOLD software. The axes are scaled by distance markers (kb). At the vertical axis, the exons (horizontal bars) and introns (vertical line) of *CD44* (NM_000610) and *OPN* (NM_001040058) are described. The significant SNPs are indicated by black (P < 0.05) or red (*P* < Bonferroni threshold; 0.05 divided by the total number of analyzed SNPs) filled circles. **a**, *CD44;* The tag SNPs, rs353647, rs112762 and rs7930724, were significant in our study (rs353647 and 112762 (black filled circles); *P* < 0.05, rs7930724 (red filled circle); *P* = 2.0 × 10⁻³; stage I + II). The rs1071695 (coding SNP; red filled circle) was identified by searching the moderate LD blocks (exons 2-13) including the significant tag SNPs, and its strong association with T2D was confirmed using the same cohorts (OR = 1.43 [95%C.I. 1.17-1.74], *P* = 3.9 × 10⁻⁴; stage I+II). The area including exon 3 has not yet been genotyped in any recent GWAS for T2D. **b,** *OPN;* The tag SNPs in 3'UTR, rs4754, rs9138 and rs10012150, were significant in our study (rs4754 and rs9138 (black filled circles); *P* < 0.05, rs10012150 (red filled circle); *P* = 1.5 × 10⁻³; stage I + II). The rs1126772 (3'UTR SNP; red filled circle) was identified by searching the LD blocks including the significant tag SNPs, and its significant association with T2D was confirmed (*P* = 4.4 × 10⁻³; stage I+II). The 3'UTR has not yet been genotyped in the recent GWAS for T2D.

### DETAILED DESCRIPTION OF THE EMBODIMENTS

Methods of diagnosing and treating an increased susceptibility to type 2 diabetes mellitus are described herein. By evaluating an individual genotype for the presence of polymorphisms in one or more of CD44, SPP1 and HDC, the susceptibility to development of T2D can be predicted. In some embodiments, the analysis is combined with determining the presence of other risk alleles, which include, without limitation, TCF7L2, KCNQ1 and the like. Diagnosis may also be made by a determination of serum levels of soluble CD44. In some embodiments, methods described herein are for decreasing the risk or for treating type 2 diabetes, by administering to an individual an inhibitor of CD44 activity.

Described herein are methods for prevention or treatment of type 2 diabetes, by administering to an individual an inhibitor of CD44 activity. Inhibitors of interest are optionally targeted through a targeting moiety, by localized drug delivery or by selectivity for a CD44 splice variant of interest. Inhibitors of interest include, without limitation, inhibitors of the interaction between the CD44 receptor and one or more of it's ligands, including osteopontin; hyaluronan; dermatan sulfate proteoglycan biglycan; serglycin; etc. In some embodiments the inhibitor is a fragment or derivative of a CD44 ligand, e.g. osteopontin peptides, soluble hyaluranan, and the like. In other embodiments, the susceptibility of the individual to developing disease is assessed prior to initiation of treatment by the methods of the invention.

The knowledge about a genetic variant that confers a risk of developing type 2 diabetes offers the opportunity to apply a genetic test to distinguish between individuals with increased risk of developing the disease and those with an average risk of developing the disease. The core values of genetic testing are the possibilities of being able to diagnose the disease at an early stage and provide information to the clinician about prognosis/aggressiveness of the disease in order to be able to apply'the most appropriate treatment. For example, the application of a genetic test for type 2 diabetes can provide an opportunity for the detection of the disease at an earlier stage which may lead to the application of therapeutic measures at an earlier stage, and thus can minimize the deleterious effects of the symptoms and serious health consequences conferred by type 2 diabetes.

### Definitions

"Activity" of CD44 shall mean any signaling or binding function performed by that protein. The CD44 protein is a cell-surface glycoprotein involved in cell-cell interactions, cell adhesion and migration. It is a receptor for hyaluronic acid and can also interact with other ligands, such as osteopontin, dermatan sulfate proteoglycan biglycan; serglycin; collagens, and matrix metalloproteinases (MMPs). CD44 participates in a wide variety of cellular functions including lymphocyte activation, recirculation and homing, hematopoiesis, and tumor metastasis. Transcripts for this gene undergo complex alternative splicing that results in many functionally distinct isoforms. Alternative splicing is the basis for the structural and functional diversity of this protein. CD44 gene transcription is at least in part activated by beta catenin and Wnt signaling.

The CD44 gene contains 19 exons spanning 50 kb of genomic DNA. The annotated genomic sequence may be accessed at Genbank, NG_008937. The CD44 gene has 10 alternatively spliced exons within the extracellular domain. In addition to the inclusion or exclusion of whole exons, additional diversity is generated through the utilization of internal splice donor and acceptor sites within 2 of the exons. A variation in the cytoplasmic domain has been shown to result from the alternative splicing of 2 exons. Thus the genomic structure of CD44 is complex, and alternative splicing is the basis of its structural and functional diversity. "CD44" shall mean the human protein encoded by the genetic sequence set forth in GenBank Accession No. NG_008937, all naturally occurring variants including splice variants and homologues thereof, and where applicable herein, all antigenic fragments thereof.

Secreted phosphoprotein 1 (SPP1), also known as bone sialoprotein I (BSP-1), early T-lymphocyte activation (ETA-1), and most commonly as osteopontin (OPN) is a single-chain polypeptide with a molecular weight of approximately 32,600. It is glycosylated (5-6 O-linked and one N-linked oligosaccharides in rat OPN), highly, but variably phosphorylated (12 phospho-Ser and one phospho-Thr in rat) and sulphated. The sequence of mRNAs encoding the human osteopontin transcripts may be accessed at Genbank, accession numbers NM_001040060; NM_001040058; and NM_000582. The gene has 7 exons, spans 5 kilobases in length and in humans it is located on the long arm of chromosome 4.

Histidine decarboxylase (HDC) is homodimeric enzyme that is a pyridoxal phosphate (PLP)-dependent decarboxylase, and is highly specific for its histidine substrate. The deduced 662-amino acid protein has a molecular mass of 74,148 Da. A high degree of homology was found among the HDCs and the dopa decarboxylases (DDC; 107930) of rat, mouse, and human. In humans the gene maps to 15q21-q22; and the sequence of the mRNA may be accessed at Genbank, NM_002112. The gene contains 12 exons spanning approximately 24 kb. Genomic DNA blot analysis suggested that HDC is encoded by a single copy gene. Structural analysis showed that the observed heterogeneity of the HDC mRNA is caused by alternative splicing.

Polymorphism, as used herein refers to variants in the gene sequence. Such variants may include single nucleotide polymorphisms, splice variants, insertions, deletions and transpositions. The polymorphisms can be those variations (DNA sequence differences) that are generally found between individuals or different ethnic groups and geographic locations which, while having a different sequence, produce functionally equivalent gene products. Polymorphisms also encompass variations which can be classified as alleles and/or mutations which can produce gene products which may have an altered function, i.e. variants in the sequence which can lead to gene products that are not functionally equivalent. Polymorphisms also encompass variations which can be classified as alleles and/or mutations which either produce no gene product, an inactive gene product or increased gene product. Further, the term is also used interchangeably with allele as appropriate.

Where a polymorphic site is a single nucleotide in length, the site is referred to as a single nucleotide polymorphism ("SNP").

As used herein, the term "nucleic acid probe" refers to a molecule capable of sequence specific hybridization to a nucleic acid, and includes analogs of nucleic acids, as are known in the art, *e.g*. DNA, RNA, peptide nucleic acids, and the like, and may be double-stranded or single-stranded. Nucleic acids can be fused to a marker sequence, for example, a sequence that encodes a polypeptide to assist in isolation or purification of the polypeptide. Such sequences include, but are not limited to, those that encode a glutathione-5-transferase (GST) fusion protein and those that encode a hemagglutinin A (HA) polypeptide marker from influenza. Other alterations of the nucleic acid molecules described herein can include, for example, labeling, methylation, internucleotide modifications such as uncharged linkages (e.g., methyl phosphonates, phosphotriesters, phosphoamidates, carbamates), charged linkages (e.g., phosphorothioates, phosphorodithioates), pendent moieties (e.g., polypeptides), intercalators (e.g., acridine, psoralen), chelators, alkylators, and modified linkages (e.g., alpha anomeric nucleic acids). Also included are synthetic molecules that mimic nucleic acid molecules in the ability to bind to a designated sequence via hydrogen bonding and other chemical interactions. Such molecules include, for example, those in which peptide linkages substitute for phosphate linkages in the backbone of the molecule.

"Specific hybridization," as used herein, refers to the ability of a first nucleic acid to hybridize to a second nucleic acid in a manner such that the first nucleic acid does not hybridize to any nucleic acid other than to the second nucleic acid. "Stringency conditions" for hybridization is a term of art which refers to the incubation and wash conditions, e.g., conditions of temperature and buffer concentration, which permit hybridization of a particular nucleic acid to a second nucleic acid; the first nucleic acid may be perfectly (i.e., 100%) complementary to the second, or the first and second may share some degree of complementarity which is less than perfect (e.g., 70%, 75%, 85%, 90%, 95%). The percent homology or identity of two nucleotide or amino acid sequences can be determined by aligning the sequences for optimal comparison purposes (e.g., gaps can be introduced in the sequence of a first sequence for optimal alignment). The nucleotides or amino acids at corresponding positions are then compared, and the percent identity between the two sequences is a function of the number of identical positions shared by the sequences (i.e., % identity=# of identical positions/total # of positions x 100). When a position in one sequence is occupied by the same nucleotide or amino acid residue as the corresponding position in the other sequence, then the molecules are homologous at that position. As used herein, nucleic acid or amino acid "homology" is equivalent to nucleic acid or amino acid "identity". A preferred, non-limiting example of such a mathematical algorithm is described in Karlin et al., Proc. Natl. Acad. Sci. USA 90:5873-5877 (1993). Such an algorithm is incorporated into the NBLAST and XBLAST programs (version 2.0) as described in Altschul et al., Nucleic Acids Res. 25:389-3402 (1997). When utilizing BLAST and Gapped BLAST programs, the default parameters of the respective programs (e.g., NBLAST) can be used. In one aspect, parameters for sequence comparison can be set at score=100, wordlength=12, or can be varied (e.g., W=5 or W=20).

Nucleic acid molecules of interest as probes are at least about 15, preferably at least about 18, 20, 23 or 25 nucleotides, and can be 30, 40, 50, 100, 200 or more nucleotides in length, and encompass the polymorphic residues of the SNP markers described herein. A probe or primer comprises a region of nucleotide sequence that hybridizes to at least about 15, for example about 20-25, and in certain aspects about 40, 50 or 75 consecutive nucleotides of a nucleic acid molecule comprising a contiguous nucleotide sequence of *CD44, SPP1* or *HDC* or polymorphic variants thereof. In other aspects, a probe or primer comprises 100 or fewer nucleotides, in certain aspects from 6 to 50 nucleotides, for example from 12 to 30 nucleotides. In other aspects, the probe or primer is at least 70% identical to the contiguous nucleotide sequence or to the complement of the contiguous nucleotide sequence, for example at least 80% identical, in certain aspects at least 90% identical, and in other aspects at least 95% identical, or even capable of selectively hybridizing to the contiguous nucleotide sequence or to the complement of the contiguous nucleotide sequence. Often, the probe or primer further comprises a label, e.g., radioisotope, fluorescent compound, enzyme, or enzyme co-factor.

A "marker", as described herein, refers to a genomic sequence characteristic of a particular allele at a polymorphic site.

SNP nomenclature as reported herein refers to the official Reference SNP (rs) ID identification tag as assigned to each unique SNP by the National Center for Biotechnological Information (NCBI).

A "haplotype," as described herein, refers to a segment of a genomic DNA strand that is characterized by a specific combination of genetic markers ("alleles") arranged along the segment. In a certain embodiment, the haplotype can comprise one or more alleles, two or more alleles, three or more alleles, four or more alleles, or five or more alleles.

The term "susceptibility", as described herein, means primarily increased susceptibility. Thus, particular markers and/or haplotypes described herein may be characteristic of increased susceptibility of type 2 diabetes, as characterized by a relative risk of greater than one. Markers and/or haplotypes that confer increased susceptibility of type 2 diabetes are furthermore considered to be "at-risk", as they confer an increased risk of disease.

"Comparable cell" shall mean a cell whose type is identical to that of another cell to which it is compared. Examples of comparable cells are cells from the same cell line.

"Inhibiting" the onset of a disorder shall mean either lessening the likelihood of the disorder's onset, or preventing the onset of the disorder entirely. In the preferred embodiment, inhibiting the onset of a disorder means preventing its onset entirely.

"Treating" a disorder shall mean slowing, stopping or reversing the disorder's progression. In the preferred embodiment, treating a disorder means reversing the disorder's progression, ideally to the point of eliminating the disorder itself. As used herein, ameliorating a disorder and treating a disorder are equivalent.

"Inhibiting" the expression of a gene in a cell shall mean either lessening the degree to which the gene is expressed, or preventing such expression entirely. "Specifically inhibit" the expression of a protein shall mean to inhibit that protein's expression (a) more than the expression of any other protein, or (b) more than the expression of all but 10 or fewer other proteins.

"Antibody" shall include, by way of example, both naturally occurring and non-naturally occurring antibodies. Specifically, this term includes polyclonal and monoclonal antibodies, and fragments thereof. Furthermore, this term includes chimeric antibodies and wholly synthetic antibodies, and fragments thereof.

"Anti-sense nucleic acid" shall mean any nucleic acid which, when introduced into a cell, specifically hybridizes to at least a portion of an mRNA in the cell encoding a protein ("target protein") whose expression is to be inhibited, and thereby inhibits the target protein's expression.

"Specifically hybridize" to a nucleic acid shall mean, with respect to a first nucleic acid, that the first nucleic acid hybridizes to a second nucleic acid with greater affinity than to any other nucleic acid.

"Subject" or "patient" shall mean any animal, such as a human, non-human primate, mouse, rat, guinea pig or rabbit.

"Suitable conditions" shall have a meaning dependent on the context in which this term is used. That is, when used in connection with an antibody, the term shall mean conditions that permit an antibody to bind to its corresponding antigen. When this term is used in connection with nucleic acid hybridization, the term shall mean conditions that permit a nucleic acid of at least 15 nucleotides in length to hybridize to a nucleic acid having a sequence complementary thereto. When used in connection with contacting an agent to a cell, this term shall mean conditions that permit an agent capable of doing so to enter a cell and perform its intended function. In one embodiment, the term "suitable conditions" as used herein means physiological conditions.

Unless otherwise apparent from the context, all elements, steps or features of the invention can be used in any combination with other elements, steps or features.

General methods in molecular and cellular biochemistry can be found in such standard textbooks as Molecular Cloning: A Laboratory Manual, 3rd Ed. (Sambrook et al., Harbor Laboratory Press 2001); Short Protocols in Molecular Biology, 4th Ed. (Ausubel et al. eds., John Wiley & Sons 1999); Protein Methods (Bollag et al., John Wiley & Sons 1996); Nonviral Vectors for Gene Therapy (Wagner et al. eds., Academic Press 1999); Viral Vectors (Kaplift & Loewy eds., Academic Press 1995); Immunology Methods Manual (I. Lefkovits ed., Academic Press 1997); and Cell and Tissue Culture: Laboratory Procedures in Biotechnology (Doyle & Griffiths, John Wiley & Sons 1998). Reagents, cloning vectors, and kits for genetic manipulation referred to in this disclosure are available from commercial vendors such as BioRad, Stratagene, Invitrogen, Sigma-Aldrich, and ClonTech.

The present invention has been described in terms of particular embodiments found or proposed by the present inventor to comprise preferred modes for the practice of the invention. It will be appreciated by those of skill in the art that, in light of the present disclosure, numerous modifications and changes can be made in the particular embodiments exemplified without departing from the intended scope of the invention. For example, due to codon redundancy, changes can be made in the underlying DNA sequence without affecting the protein sequence. Moreover, due to biological functional equivalency considerations, changes can be made in protein structure without affecting the biological action in kind or amount. All such modifications are intended to be included within the scope of the appended claims.

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Methods of Treatment Using CD44 specific Inhibitors

Methods described herein include administering to an individual having type 2 diabetes, or diagnosed as susceptible to development of type 2 diabetes (e.g. diagnosed by the methods of the invention described herein) an inhibitor of CD44 activity. The inhibitor may be selective for a splice variant of CD44, for example a splice variant that includes, or is targeted to, exon 3 of CD44. Administration may be systemic or localized, where the dosage and timing of administration is determined as described herein.

An inhibitory agent may inhibit the activity of CD44 by a variety of different mechanisms. In certain embodiments, the inhibitory agent is one that binds to the CD44 extracellular domain and, in doing so, inhibits its ligand binding activity. In other embodiments, the inhibitory agent prevents expression or secretion of CD44. In other embodiments, the inhibitory agent binds to the cytoplasmic domain of CD44 and interferes with signaling pathways mediated by CD44.

Representative CD44 inhibitory agents include, but are not limited to: antisense oligonucleotides; antibodies; fragments, particularly soluble fragments of CD44 ligands, small molecules, and the like. Naturally occurring or synthetic small molecule compounds of interest include numerous chemical classes, though typically they are organic molecules, preferably small organic compounds having a molecular weight of more than 50 and less than about 2,500 daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof. Such molecules may be identified, among other ways, by employing appropriate screening protocols.

The inhibitory agent may act on CD44 mRNA to inhibit the activity of the target CD44 by reducing the amount of CD44 RNA present in the targeted cells. By "reducing the amount of" is meant that the level or quantity of the target CD44 mRNA in the target cell is reduced by at least about 2-fold, usually by at least about 5-fold, e.g., 10-fold, 15-fold, 20-fold, 50-fold, 100-fold or more, as compared to a control, i.e., an identical target cell not treated according to the subject methods. Such inhibitors include RNAi and antisense oligonucleotides; which may be selective for a specific CD44 splice variant of interest.

An antisense reagent may be an antisense oligonucleotide (ODN), particularly synthetic ODN having chemical modifications from native nucleic acids, or nucleic acid constructs that express such antisense molecules as RNA. The antisense sequence is complementary to the targeted mRNA, and inhibits its expression. One or a combination of antisense molecules may be administered, where a combination may comprise multiple different sequences.

Antisense molecules may be produced by expression of all or a part of the target CD44 sequence in an appropriate vector, where the transcriptional initiation is oriented such that an antisense strand is produced as an RNA molecule. Alternatively, the antisense molecule is a synthetic oligonucleotide. Antisense oligonucleotides will generally be at least about 7, usually at least about 12, more usually at least about 20 nucleotides in length, and not more than about 25, usually not more than about 23-22 nucleotides in length, where the length is governed by efficiency of inhibition, specificity, including absence of cross-reactivity, and the like.

Antisense oligonucleotides may be chemically synthesized by methods known in the art (see Wagner *et al.* (1993) supra. and Milligan *et al.,* supra.) Preferred oligonucleotides are chemically modified from the native phosphodiester structure, in order to increase their intracellular stability and binding affinity. A number of such modifications have been described in the literature that alter the chemistry of the backbone, sugars or heterocyclic bases.

Among useful changes in the backbone chemistry are phosphorothioates; phosphorodithioates, where both of the non-bridging oxygens are substituted with sulfur; phosphoroamidites; alkyl phosphotriesters and boranophosphates. Achiral phosphate derivatives include 3'-O'-5'-S-phosphorothioate, 3'-S-5'-O-phosphorothioate, 3'-CH2-5'-O-phosphonate and 3'-NH-5'-O-phosphoroamidate. Peptide nucleic acids replace the entire ribose phosphodiester backbone with a peptide linkage. Sugar modifications are also used to enhance stability and affinity. The α-anomer of deoxyribose may be used, where the base is inverted with respect to the natural β-anomer. The 2'-OH of the ribose sugar may be altered to form 2'-O-methyl or 2'-O-allyl sugars, which provides resistance to degradation without comprising affinity. Modification of the heterocyclic bases must maintain proper base pairing. Some useful substitutions include deoxyuridine for deoxythymidine; 5-methyl-2'-deoxycytidine and 5-bromo-2'-deoxycytidine for deoxycytidine. 5-propynyl-2'-deoxyuridine and 5-propynyl-2'-deoxycytidine have been shown to increase affinity and biological activity when substituted for deoxythymidine and deoxycytidine, respectively.

Anti-sense molecules of interest include antagomir RNAs, *e.g*. as described by Krutzfeldt *et al., supra.* Small interfering double-stranded RNAs (siRNAs) engineered with certain 'drug-like' properties such as chemical modifications for stability and cholesterol conjugation for delivery have been shown to achieve therapeutic silencing of an endogenous gene *in vivo.* To develop a pharmacological approach for silencing mRNAs *in vivo,* chemically modified, cholesterol-conjugated single-stranded RNA analogues complementary to mRNAs were developed, termed 'antagomirs'. Antagomir RNAs may be synthesized using standard solid phase oligonucleotide synthesis protocols. The RNAs are conjugated to cholesterol, and may further have a phosphorothioate backbone at one or more positions.

Also of interest in certain embodiments are RNAi agents. By RNAi agent is meant an agent that modulates expression of mRNA by a RNA interference mechanism. The RNAi agents employed in one embodiment described herein are small ribonucleic acid molecules (also referred to herein as interfering ribonucleic acids), i.e., oligoribonucleotides, that are present in duplex structures, e.g., two distinct oligoribonucleotides hybridized to each other or a single ribooligonucleotide that assumes a small hairpin formation to produce a duplex structure. By oligoribonucleotide is meant a ribonucleic acid that does not exceed about 100 nt in length, and typically does not exceed about 75 nt length, where the length in certain embodiments is less than about 70 nt. Where the RNA agent is a duplex structure of two distinct ribonucleic acids hybridized to each other, e.g., an siRNA, the length of the duplex structure typically ranges from about 15 to 30 bp, usually from about 15 to 29 bp, where lengths between about 20 and 29 bps, e.g., 21 bp, 22 bp, are of particular interest in certain embodiments. Where the RNA agent is a duplex structure of a single ribonucleic acid that is present in a hairpin formation, i.e., a shRNA, the length of the hybridized portion of the hairpin is typically the same as that provided above for the siRNA type of agent or longer by 4-8 nucleotides. The weight of the RNAi agents of this embodiment typically ranges from about 5,000 daltons to about 35,000 daltons, and in many embodiments is at least about 10,000 daltons and less than about 27,500 daltons, often less than about 25,000 daltons.

dsRNA can be prepared according to any of a number of methods that are known in the art, including in vitro and in vivo methods, as well as by synthetic chemistry approaches. Examples of such methods include, but are not limited to, the methods described by Sadher *et al.* (Biochem. Int. 14:1015, 1987); by Bhattacharyya (Nature 343:484, 1990); and by Livache, *et al.* (U.S. Patent No. 5,795,715). Single-stranded RNA can also be produced using a combination of enzymatic and organic synthesis or by total organic synthesis. The use of synthetic chemical methods enable one to introduce desired modified nucleotides or nucleotide analogs into the dsRNA. dsRNA can also be prepared in vivo according to a number of established methods (see, e.g., Sambrook, et al. (1989) Molecular Cloning: A Laboratory Manual, 2nd ed.; Transcription and Translation (B.D. Hames, and S.J. Higgins, Eds., 1984); DNA Cloning, volumes I and II (D.N. Glover, Ed., 1985); and Oligonucleotide Synthesis (M.J. Gait, Ed., 1984).

In certain embodiments, instead of the RNAi agent being an interfering ribonucleic acid, e.g., an siRNA or shRNA as described above, the RNAi agent may encode an interfering ribonucleic acid, e.g., an shRNA, as described above. In other words, the RNAi agent may be a transcriptional template of the interfering ribonucleic acid. In these embodiments, the transcriptional template is typically a DNA that encodes the interfering ribonucleic acid. The DNA may be present in a vector, where a variety of different vectors are known in the art, e.g., a plasmid vector, a viral vector, etc.

In another embodiment, the CD44 inhibitor is an antibody. The term "antibody" or "antibody moiety" is intended to include any polypeptide chain-containing molecular structure with a specific shape that fits to and recognizes an epitope, where one or more non-covalent binding interactions stabilize the complex between the molecular structure and the epitope. The term includes monoclonal antibodies, multispecific antibodies (antibodies that include more than one domain specificity), human antibody, humanized antibody, and antibody fragments with the desired biological activity.

Polyclonal antibodies can be raised by a standard protocol by injecting a production animal with an antigenic composition, formulated as described above. See, e.g., Harlow and Lane, Antibodies: A Laboratory Manual, Cold Spring Harbor Laboratory, 1988. In one such technique, a Class II target antigen comprising an antigenic portion of the polypeptide is initially injected into any of a wide variety of mammals (e.g., mice, rats, rabbits, sheep or goats). When utilizing an entire protein, or a larger section of the protein, antibodies may be raised by immunizing the production animal with the protein and a suitable adjuvant (e.g., Freund's, Freund's complete, oil-in-water emulsions, etc.) Alternatively, for monoclonal antibodies, hybridomas may be formed by isolating the stimulated immune cells, such as those from the spleen of the inoculated animal. These cells are then fused to immortalized cells, such as myeloma cells or transformed cells, which are capable of replicating indefinitely in cell culture, thereby producing an immortal, immunoglobulin-secreting cell line.

In addition, the antibodies or antigen binding fragments may be produced by genetic engineering. In this technique, as with the standard hybridoma procedure, antibody-producing cells are sensitized to the desired antigen or immunogen. The messenger RNA isolated from the immune spleen cells or hybridomas is used as a template to make cDNA using PCR amplification. A library of vectors, each containing one heavy chain gene and one light chain gene retaining the initial antigen specificity, is produced by insertion of appropriate sections of the amplified immunoglobulin cDNA into the expression vectors. A combinatorial library is constructed by combining the heavy chain gene library with the light chain gene library. This results in a library of clones, which co-express a heavy and light chain (resembling the Fab fragment or antigen binding fragment of an antibody molecule). The vectors that carry these genes are co-transfected into a host (e.g. bacteria, insect cells, mammalian cells, or other suitable protein production host cell). When antibody gene synthesis is induced in the transfected host, the heavy and light chain proteins self-assemble to produce active antibodies that can be detected by screening with the antigen or immunogen.

Antibodies with a reduced propensity to induce a violent or detrimental immune response in humans (such as anaphylactic shock), and which also exhibit a reduced propensity for priming an immune response which would prevent repeated dosage with the antibody therapeutic are preferred for use in the invention. Thus, humanized, single chain, chimeric, or human antibodies, which produce less of an immune response when administered to humans, are preferred for use in the present invention. Also included in the invention are multi-domain antibodies.

A chimeric antibody is a molecule in which different portions are derived from different animal species, for example those having a variable region derived from a murine mAb and a human immunoglobulin constant region. Techniques for the development of chimeric antibodies are described in the literature. See, for example, Morrison et al. (1984) Proc. Natl. Acad. Sci. 81:6851-6855; Neuberger et al. (1984) Nature 312:604-608; Takeda et al. (1985) Nature 314:452-454. Single chain antibodies are formed by linking the heavy and light chain fragments of the Fv region via an amino acid bridge, resulting in a single chain polypeptide. See, for example, Huston et al., Science 242:423-426; Proc. Natl. Acad. Sci. 85:5879-5883; and Ward et al. Nature 341:544-546.

Antibody fragments that recognize specific epitopes may be generated by techniques well known in the field. These fragments include, without limitation, F(ab')₂ fragments, which can be produced by pepsin digestion of the antibody molecule, and Fab fragments, which can be generated by reducing the disulfide bridges of the F(ab')₂ fragments.

Alternatively, single chain antibodies (Fv, as described below) can be produced from phage libraries containing human variable regions. See U.S. Patent No. 6,174,708. Intrathecal administration of single-chain immunotoxin, LMB-7 [B3(Fv)- PE38], has been shown to cure of carcinomatous meningitis in a rat model. Proc Natl. Acad. Sci U S A 92, 2765-9.

In addition to entire immunoglobulins (or their recombinant counterparts), immunoglobulin fragments comprising the epitope binding site (e.g., Fab', F(ab')₂, or other fragments) are useful as antibody moieties in the present invention. Such antibody fragments may be generated from whole immunoglobulins by ficin, pepsin, papain, or other protease cleavage. "Fragment," or minimal immunoglobulins may be designed utilizing recombinant immunoglobulin techniques. For instance "Fv" immunoglobulins for use in the present invention may be produced by linking a variable light chain region to a variable heavy chain region via a peptide linker (e.g., poly-glycine or another sequence which does not form an alpha helix or beta sheet motif).

Candidate antibodies can be tested for by any suitable standard means, *e.g*. ELISA assays, *etc.* As a first screen, the antibodies may be tested for binding against the immunogen. After selective binding is established, the candidate antibody may be tested for appropriate activity in an *in vivo* model. In a preferred embodiment, antibody compounds may be screened using a variety of methods *in vitro* and *in vivo.* These methods include, but are not limited to, methods that measure binding affinity to a target, biodistribution of the compound within an animal or cell, or compound mediated cytotoxicity. These and other screening methods known in the art provide information on the ability of a compound to bind to, modulate, or otherwise interact with the specified target and are a measure of the compound's efficacy.

Anti-CD44 antibodies may be administered daily, semi-weekly, weekly, semi-monthly, monthly, etc., at a dose of from about 0.01 mg, from about 0.1 mg, from about 1 mg, from about 5 mg, from about 10 mg, from about 100 mg or more per kilogram of body weight when administered systemically. Smaller doses may be utilized in localized administration, e.g. in direct administration to ocular nerves, *etc.* Humanized, chimeric human, or human antibodies are preferred for administering to human patients.

In other embodiments described herein the inhibitor is a derivative of a CD44 ligand, which acts to block activity, *e.g*. a soluble ligand, a ligand analog, a fragment of a ligand, *etc.* Molecules of interest include the pentapeptide GRGDS, endotoxin-free low molecular weight hyaluronan; and hyaluronic acid esterified with butyric acid residues. The inhibitor, F-19848 A inhibits the binding of CD44 and HA with an IC50 value of 23.5 µM in a cell-based assay (Hirota-Takahata (2007) J Antibiot 60(10):633-9.

In other embodiments the inhibitor is a drug that inhibits CD44 activity, particularly a small molecule drug. Such compounds may be those known in the art, or may be developed using the screening methods described herein.

Determining a therapeutically or prophylactically effective amount of the CD44 inhibitor compositions can be done based on animal data using routine computational methods. In one embodiment, the therapeutically or prophylactically effective amount contains between about 0.01 mg and about 1 g of inhibitor, e.g. nucleic acid, protein or peptide, *etc.,* as applicable. In another embodiment, the effective amount contains between about 1 mg and about 100 mg of inhibitor, as applicable. In a further embodiment, the effective amount contains between about 10 mg and about 50 mg of the inhibitor, as applicable.

Administering the instant compositions can be effected or performed using any of the various methods and delivery systems known to those skilled in the art. The administering can be performed, for example, intravenously, orally, via implant, transmucosally, transdermally, intramuscularly, intrathecally, and subcutaneously. The following delivery systems, which employ a number of routinely used pharmaceutical carriers, are only representative of the many embodiments envisioned for administering the instant compositions.

Injectable drug delivery systems include solutions, suspensions, gels, microspheres and polymeric injectables, and can comprise excipients such as solubility-altering agents (e.g., ethanol, propylene glycol and sucrose) and polymers (e.g., polycaprylactones and PLGA's). Implantable systems include rods and discs, and can contain excipients such as PLGA and polycaprylactone. Nucleic acids described herein can also be administered attached to particles using a gene gun.

Oral delivery systems include tablets and capsules. These can contain excipients such as binders (e.g., hydroxypropylmethylcellulose, polyvinyl pyrilodone, other cellulosic materials and starch), diluents (e.g., lactose and other sugars, starch, dicalcium phosphate and cellulosic materials), disintegrating agents (e.g., starch polymers and cellulosic materials) and lubricating agents (e.g., stearates and talc).

Transmucosal delivery systems include patches, tablets, suppositories, pessaries, gels and creams, and can contain excipients such as solubilizers and enhancers (e.g., propylene glycol, bile salts and amino acids), and other vehicles (e.g., polyethylene glycol, fatty acid esters and derivatives, and hydrophilic polymers such as hydroxypropylmethylcellulose and hyaluronic acid).

Dermal delivery systems include, for example, aqueous and nonaqueous gels, creams, multiple emulsions, microemulsions, liposomes, ointments, aqueous and nonaqueous solutions, lotions, aerosols, hydrocarbon bases and powders, and can contain excipients such as solubilizers, permeation enhancers (e.g., fatty acids, fatty acid esters, fatty alcohols and amino acids), and hydrophilic polymers (e.g., polycarbophil and polyvinylpyrolidone). In one embodiment, the pharmaceutically acceptable carrier is a liposome or a transdermal enhancer.

Solutions, suspensions and powders for reconstitutable delivery systems include vehicles such as suspending agents (e.g., gums, xanthans, cellulosics and sugars), humectants (e.g., sorbitol), solubilizers (e.g., ethanol, water, PEG and propylene glycol), surfactants (e.g., sodium lauryl sulfate, Spans, Tweens, and cetyl pyridine), preservatives and Jun. *2,2005* antioxidants (e.g., parabens, vitamins E and C, and ascorbic acid), anti-caking agents, coating agents, and chelating agents (e.g., EDTA).

### Diagnostic Methods

Described herein are genetic variants in the *CD44, SPP1* and *HDC* genes that are genetic markers for an individual's susceptibility to type 2 diabetes mellitus (T2D). In diagnostic methods described herein, an individual may be evaluated for the presence of polymorphisms in one or more of *CD44, SPP1* and *HDC;* and may further be evaluated for one or more polymorphisms within each of these genetic loci. Thus an individual may be genotyped for the presence of one or two alleles in one, two, three, four, five or more different polymorphic loci set forth herein. In some embodiments the polymorphism is a single nucleotide polymorphism (SNP).

Assessment of risk may also include analysis of genomic regions contiguous with the genetic polymorphisms described herein. Large regions of DNA are often inherited as block and, as such, contain causal as well as non-causal polymorphisms in close proximity. Thus a polymorphism may be inherited as a "linkage disequilibrium block" or "LD block" together with numerous other polymorphisms.

In one embodiment described herein, diagnosis of a susceptibility to type 2 diabetes is carried out by detecting a predisposing polymorphism in one or more of the CD44, SPP1 and HDC loci. The polymorphism can be a change in a *CD44, SPP1* and/or *HDC* nucleic acid, such as the insertion or deletion of a single nucleotide, or of more than one nucleotide, resulting in a frame shift; the change of at least one nucleotide, resulting in a change in the encoded amino acid; the change of at least one nucleotide, resulting in the generation of a premature stop codon; the deletion of several nucleotides, resulting in a deletion of one or more amino acids encoded by the nucleotides; the insertion of one or several nucleotides, such as by unequal recombination or gene conversion, resulting in an interruption of the coding sequence of the gene; duplication of all or a part of the gene; transposition of all or a part of the gene; alternation is the splicing, or rearrangement of all or a part of the gene. More than one such change may be present in a single gene.

Such sequence changes may cause a difference in the polypeptide encoded by a CD44, SPP1 or HDC nucleic acid. For example, if the difference is a frame shift change, the frame shift can result in a change in the encoded amino acids, and/or can result in the generation of a premature stop codon, causing generation of a truncated polypeptide. Alternatively, a polymorphism associated with a disease or condition or a susceptibility to a disease or condition associated with a *CD44, SPP1* or *HDC* nucleic acid can be a synonymous alteration in one or more nucleotides (i.e., an alteration that does not result in a change in the polypeptide encoded by a *CD44, SPP1* or *HDC* nucleic acid). Such a polymorphism may alter splicing sites, affect the stability or transport of mRNA, or otherwise affect the transcription or translation of the gene. A *CD44, SPP1* or *HDC* nucleic acid that has any of the changes or alterations described above is referred to herein as an "altered nucleic acid."

Specific polymorphisms of interest include those present in an SNP of the CD44 locus, *e.g*. the attached table of CD44 polymorphisms. In some embodiments of the invention, an individual is genotyped by one or more CD44 SNP sequences selected from rs353647; rs112762; rs7930724; rs1071695. SNP rs1071695, which is located in exon 3 of CD44, is of interest, and finds particular use in the genotyping of individuals of Japanese ethnicity. The sequence at the site of the polymorphisms is as follows, where the risk allele is underlined:

| | | |
|---|---|---|
| SEQ ID NO:1 | rs353647 | CAATGTCATCCAAGTGGGAAAGTCAG[C/G]ATTCTAACCTCTATGCTATGT TGCC |
| SEQ ID NO:2 | rs 112762 | GCCTAAAATAGAGCCTGGCATATACA[C/T]AATGAATGCACAATAAATATT AGCC |
| SEQ ID NO:3 | rs1071695 | TACAGGTATGGGTTCATAGAAGGGCA[C/T]GTGGTGATTCCCCGGATCCAC CCCA |
| SEQ ID NO:4 | rs7930724 | ATTTACTCAACAATTCCTTTGTTTTT[A/G]GTCCAAGGAAGCATATCTGGA AGAG |

Specific polymorphisms of SPP1 (osteopontin) include, without limitation SNPs rs4754; rs9138; rs10012150; rs1126616 and rs1126772. The sequence at the site of the polymorphism is as follows, where the risk allele is underlined:

| | | |
|---|---|---|
| SEQ ID NO:5 | rs4754 | GATGATATGGATGATGAAGATGATGA[C/T]GACCATGTGGACAGCCAGGAC TCCA |
| SEQ ID NO:6 | rs9138 | CTCTCATGAATAGAAATTTATGTAGA[A/C]GCAAACAAAATACTTTTACCC ACTT |
| SEQ ID NO:7 | rs10012150 | AGCTAGAAAAGTGGATCACATAGAGA[C/T]AGACTGTAAATTGGTGGTTCT ACAA |
| SEQ ID NO:8 | rs1126616 | CAGTCCAGATTATATAAGCGGAAAGC[C/T]AATGATGAGAGCAATGAGCAT TCCG |
| SEQ ID NO:9 | rs1126772 | GTATCTATTTGAGTCTGGAAATAACT[A/G]ATGTGTTTGATAATTAGTTTA GTTT |

Specific polymorphisms of HDC include, without limitation SNPs rs854150; rs2853766; and rs2238292. The sequence at the site of the polymorphism is as follows, where the risk allele is underlined:

| | | |
|---|---|---|
| SEQ ID NO:10 | rs854150 | CTCAAGGTAAAGAAGAGCCCTGTCAG[C/G]CTTTATACGTCCAGATTTT TTGATC |
| SEQ ID NO:11 | rs2853766 | TGCCCTAGCTTACACAGCTGATAAGC[A/G]GAAGAACGAGGATTCAAAC TGATGC |
| SEQ ID NO:12 | rs2238292 | CTGCCCACTCAGCTCCTCCTTACATC[A/C]GGTTTTGATTGTGGGCTCA GGGCTT |

For determining a susceptibility to type 2 diabetes, hybridization methods, such as Southern analysis, Northern analysis, or *in situ* hybridizations, can be used (see Current Protocols in Molecular Biology, Ausubel, F. et al., eds, John Wiley & Sons, including all supplements through 1999). For example, a biological sample (a "test sample") from a test subject (the "test individual") of genomic DNA, RNA, or cDNA, is obtained from an individual (RNA and cDNA can only be used for exonic markers), such as an individual suspected of having, being susceptible to or predisposed for, or carrying a defect for, type 2 diabetes. The individual can be an adult, child, or fetus. The test sample can be from any source which contains genomic DNA, such as a blood sample, sample of amniotic fluid, sample of cerebrospinal fluid, or tissue sample from skin, muscle, buccal or conjunctival mucosa, placenta, gastrointestinal tract or other organs.

The DNA, RNA, or cDNA sample is then examined to determine which allele of a polymorphism is present in a *CD44, SPP1* and/or *HDC* nucleic acid. The presence of the allele of interest can be indicated by hybridization of the gene in the genomic DNA, RNA, or cDNA to a nucleic acid probe. A "nucleic acid probe", as used herein, can be a DNA probe or an RNA probe; the nucleic acid probe can contain, for example, at least one polymorphic residue in a *CD44, SPP1* and/or *HDC* nucleic acid. The probe can be any of the nucleic acid molecules described above (e.g., the gene or nucleic acid, a fragment, a vector comprising the gene or nucleic acid, a probe or primer, *etc.*)

A preferred probe for detecting mRNA or genomic DNA is a labeled nucleic acid probe capable of hybridizing to mRNA or genomic DNA sequences described herein. The nucleic acid probe can be, for example, a full-length nucleic acid molecule, or a portion thereof, such as an oligonucleotide of at least 15, 30, 50, 100, 250 or 500 nucleotides in length and sufficient to specifically hybridize under stringent conditions to appropriate mRNA or genomic DNA, and to distinguish between the risk allele and the protective allele.

The hybridization sample is maintained under conditions that are sufficient to allow specific hybridization of the nucleic acid probe to a *CD44, SPP1* and/or *HDC* nucleic acid. "Specific hybridization", as used herein, indicates exact hybridization (e.g., with no mismatches). Specific hybridization can be performed under high stringency conditions or moderate stringency conditions, for example, as described above. In a particularly preferred aspect, the hybridization conditions for specific hybridization are high stringency appropriate to the length of the probe.

Specific hybridization, if present, is then detected using standard methods. More than one nucleic acid probe can also be used concurrently in this method. Specific hybridization of any one of the nucleic acid probes to an allele indicated herein as being a predisposing allele is diagnostic for a susceptibility to type 2 diabetes.

In another method described herein, alteration analysis by restriction digestion can be used to detect an alteration in the gene, if the alteration (mutation) or polymorphism in the gene results in the creation or elimination of a restriction site. A test sample containing genomic DNA is obtained from the individual. Polymerase chain reaction (PCR) can be used to amplify a *CD44, SPP1* or *HDC* nucleic acid (and, if necessary, the flanking sequences) in the test sample of genomic DNA from the test individual. RFLP analysis is conducted as described (see Current Protocols in Molecular Biology). The digestion pattern of the relevant DNA fragment indicates the presence or absence of the allele indicated herein as being a predisposing allele in the *CD44, SPP1* or *HDC* nucleic acid, and therefore indicates the presence or absence of a susceptibility to type 2 diabetes.

Sequence analysis can also be used to detect specific polymorphisms in a *CD44, SPP1* or *HDC* nucleic acid. A test sample of DNA or RNA is obtained from the test individual. PCR or other appropriate methods can be used to amplify the gene or nucleic acid, and/or its flanking sequences, if desired. The sequence of a *CD44, SPP1* or *HDC* nucleic acid, or a fragment of the nucleic acid, or cDNA, or fragment of the cDNA, or mRNA, or fragment of the mRNA, is determined, using standard methods. The sequence of the nucleic acid, nucleic acid fragment, cDNA, cDNA fragment, mRNA, or mRNA fragment is compared with the known nucleic acid sequence of the gene or cDNA or mRNA, as appropriate. The presence of a polymorphism indicated herein as being a predisposing allele in the *CD44, SPP1* or *HDC* gene indicates that the individual has a susceptibility to type 2 diabetes.

Allele-specific oligonucleotides can also be used to detect the presence of a polymorphism in a *CD44, SPP1* or *HDC* nucleic acid, through the use of dot-blot hybridization of amplified oligonucleotides with allele-specific oligonucleotide (ASO) probes (see, for example, Saiki, R. et al., Nature 324:163-166 (1986)). An "allele-specific oligonucleotide" (also referred to herein as an "allele-specific oligonucleotide probe") is an oligonucleotide of approximately 10-50 base pairs that specifically hybridizes to a *CD44, SPP1* or *HDC* nucleic acid, and that contains a polymorphism associated with a susceptibility to type 2 diabetes. An allele-specific oligonucleotide probe that is specific for particular polymorphisms in a *CD44, SPP1* or *HDC* nucleic acid can be prepared, using standard methods (see Current Protocols in Molecular Biology). Described herein are allele-specific oligonucleotides that hybridize to the reference or variant allele of a gene or nucleic acid comprising a polymorphism or to the complement thereof. These oligonucleotides can be probes or primers. With the addition of such analogs as locked nucleic acids (LNAs), the size of primers and probes can be reduced to as few as 8 bases.

A test sample of DNA is obtained from the individual. PCR can be used to amplify all or a fragment of a *CD44, SPP1* or *HDC* nucleic acid and its flanking sequences. The DNA containing the amplified *CD44, SPP1* or *HDC* nucleic acid (or fragment of the gene or nucleic acid) is dot-blotted, using standard methods (see Current Protocols in Molecular Biology), and the blot is contacted with the respective oligonucleotide probe. The presence of specific hybridization of the probe to the amplified *CD44, SPP1* or *HDC* nucleic acid is then detected. Hybridization of an allele-specific oligonucleotide probe to DNA from the individual is indicative of the presence of an allele indicated herein as being a predisposing allele in the *CD44, SPP1* or *HDC* nucleic acid, and is therefore indicative of susceptibility to type 2 diabetes.

An allele-specific primer hybridizes to a site on target DNA overlapping a polymorphism and only primes amplification of an allelic form to which the primer exhibits perfect complementarity. See Gibbs, Nucleic Acid Res. 17, 2427-2448 (1989). This primer is used in conjunction with a second primer, which hybridizes at a distal site. Amplification proceeds from the two primers, resulting in a detectable product, which indicates the particular allelic form is present. A control is usually performed with a second pair of primers, one of which shows a single base mismatch at the polymorphic site and the other of which exhibits perfect complementarity to a distal site. The single-base mismatch prevents amplification and no detectable product is formed. The method works best when the mismatch is included in the 3'-most position of the oligonucleotide aligned with the polymorphism because this position is most destabilizing to elongation from the primer (see, e.g., WO 93/22456).

In another aspect, arrays of oligonucleotide probes that are complementary to target nucleic acid sequence segments from an individual can be used to identify the presence of polymorphic alleles in a *CD44, SPP1* or *HDC* nucleic acid. For example, in one aspect, an oligonucleotide array can be used. Oligonucleotide arrays typically comprise a plurality of different oligonucleotide probes that are coupled to a surface of a substrate in different known locations. These oligonucleotide arrays, also described as "Genechips™," have been generally described in the art, for example, U.S. Pat. No. 5,143,854 and PCT patent publication Nos. WO 90/15070 and 92/10092. These arrays can generally be produced using mechanical synthesis methods or light directed synthesis methods that incorporate a combination of photolithographic methods and solid phase oligonucleotide synthesis methods. See Fodor et al., Science 251:767-777 (1991), Pirrung et al., U.S. Pat. No. 5,143,854 (see also PCT Application No. WO 90/15070) and Fodor et al., PCT Publication No. WO 92/10092 and U.S. Pat. No. 5,424,186. Techniques for the synthesis of these arrays using mechanical synthesis methods are described in, e.g., U.S. Pat. No. 5,384,261. In another example, linear arrays can be utilized.

Once an oligonucleotide array is prepared, a nucleic acid of interest is hybridized with the array and scanned for polymorphisms. Hybridization and scanning are generally carried out by methods described herein and also in, e.g., published PCT Application Nos. WO 92/10092 and WO 95/11995, and U.S. Pat. No. 5,424,186. In brief, a target nucleic acid sequence that includes one or more previously identified polymorphic markers is amplified by well-known amplification techniques, e.g., PCR. Typically, this involves the use of primer sequences that are complementary to the two strands of the target sequence both upstream and downstream from the polymorphism. Asymmetric PCR techniques may also be used. Amplified target, generally incorporating a label, is then hybridized with the array under appropriate conditions. Upon completion of hybridization and washing of the array, the array is scanned to determine the position on the array to which the target sequence hybridizes. The hybridization data obtained from the scan is typically in the form of fluorescence intensities as a function of location on the array.

Additional uses of oligonucleotide arrays for polymorphism detection can be found, for example, in U.S. Pat. Nos. 5,858,659 and 5,837,832. Other methods of nucleic acid analysis can be used to detect polymorphic alleless in a type 2 diabetes gene or variants encoded by a type 2 diabetes gene. Representative methods include direct manual sequencing (Church and Gilbert, Proc. Natl. Acad. Sci. USA 81:1991-1995 (1988); Sanger, F. et al., Proc. Natl. Acad. Sci. USA 74:5463-5467 (1977); Beavis et al., U.S. Pat. No. 5,288,644); automated fluorescent sequencing; single-stranded conformation polymorphism assays (SSCP); clamped denaturing gel electrophoresis (CDGE); denaturing gradient gel electrophoresis (DGGE) (Sheffield, V. C. et al., Proc. Natl. Acad. Sci. USA 86:232-236 (1989)), mobility shift analysis (Orita, M. et al., Proc. Natl. Acad. Sci. USA 86:2766-2770 (1989)), restriction enzyme analysis (Flavell et al., Cell 15:25 (1978); Geever, et al., Proc. Natl. Acad. Sci. USA 78:5081 (1981)); heteroduplex analysis; chemical mismatch cleavage (CMC) (Cotton et al., Proc. Natl. Acad. Sci. USA 85:4397-4401 (1985)); RNase protection assays (Myers, R. M. et al., Science 230:1242 (1985)); use of polypeptides which recognize nucleotide mismatches, such as E. coli mutS protein; allele-specific PCR, for example.

Described herein is diagnosis of a susceptibility to type 2 diabetes by expression analysis by quantitative PCR (kinetic thermal cycling). This technique, utilizing TagMan^{®} assays, can assess the presence of an alteration in the expression or composition of a *CD44, SPP1* or *HDC* nucleic acid or splicing variants encoded by a *CD44, SPP1* or *HDC* nucleic acid. TaqMan^{®} probes can also be used to allow the identification of polymorphisms and whether a patient is homozygous or heterozygous. Further, the expression of the variants can be quantified as physically or functionally different.

Described herein is diagnosis of a susceptibility to type 2 diabetes by examining expression and/or composition of a *CD44, SPP1* or *HDC* polypeptide, by a variety of methods, including enzyme linked immunosorbent assays (ELISAs), Western blots, immunoprecipitations and immunofluorescence. A test sample from an individual is assessed for the presence of an alteration in the expression and/or an alteration in composition of the polypeptide encoded by a *CD44, SPP1* or *HDC* nucleic acid, or for the presence of a particular variant encoded by a *CD44, SPP1* or *HDC* nucleic acid. An alteration in expression of a polypeptide encoded by a *CD44, SPP1* or *HDC* nucleic acid can be, for example, an alteration in the quantitative polypeptide expression (i.e., the amount of polypeptide produced); an alteration in the composition of a polypeptide encoded by a *CD44, SPP1* or *HDC* nucleic acid is an alteration in the qualitative polypeptide expression (e.g., expression of an altered *CD44, SPP1* or *HDC* polypeptide or of a different splicing variant). In a preferred aspect, diagnosis of a susceptibility to type 2 diabetes can be made by detecting a particular splicing variant encoded by that *CD44, SPP1* or *HDC* nucleic acid, or a particular pattern of splicing variants.

Both such alterations (quantitative and qualitative) can also be present. The term "alteration" in the polypeptide expression or composition, as used herein, refers to an alteration in expression or composition in a test sample, as compared with the expression or composition of polypeptide by a *CD44, SPP1* or *HDC* in a control sample. A control sample is a sample that corresponds to the test sample (e.g., is from the same type of cells), and is from an individual who is not affected by a susceptibility to type II diabetes. An alteration in the expression or composition of the polypeptide in the test sample, as compared with the control sample, is indicative of a susceptibility to type 2 diabetes. Similarly, the presence of one or more different splicing variants in the test sample, or the presence of significantly different amounts of different splicing variants in the test sample, as compared with the control sample, is indicative of a susceptibility to type 2 diabetes.

Various means of examining expression or composition of the polypeptide encoded by a *CD44, SPP1* or *HDC* nucleic acid can be used, including: spectroscopy, colorimetry, electrophoresis, isoelectric focusing, and immunoassays (e.g., David et al., U.S. Pat. No. 4,376,110) such as immunoblotting (see also Current Protocols in Molecular Biology). For example, in one aspect, an antibody capable of binding to the polypeptide (e.g., as described above), preferably an antibody with a detectable label, can be used. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling a DNA probe with biotin such that it can be detected with fluorescently labeled streptavidin.

Western blotting analysis, using an antibody that specifically binds to a polypeptide encoded by an altered *CD44, SPP1* or *HDC* nucleic acid or an antibody that specifically binds to a polypeptide encoded by a non-altered nucleic acid, or an antibody that specifically binds to a particular splicing variant encoded by a nucleic acid, can be used to identify the presence in a test sample of a particular splicing variant or of a polypeptide encoded by a polymorphic or altered *CD44, SPP1* or *HDC* nucleic acid, or the absence in a test sample of a particular splicing variant or of a polypeptide encoded by a non-polymorphic or non-altered nucleic acid. The presence of a polypeptide encoded by a polymorphic or altered nucleic acid, or the absence of a polypeptide encoded by a non-polymorphic or non-altered nucleic acid, is diagnostic for a susceptibility to type 2 diabetes, as is the presence (or absence) of particular splicing variants encoded by the *CD44, SPP1* or *HDC* nucleic acid.

In one aspect of this method, the level or amount of polypeptide encoded by a *CD44, SPP1* or *HDC* nucleic acid in a test sample is compared with the level or amount of the polypeptide encoded by the *CD44, SPP1* or *HDC* in a control sample. A level or amount of the polypeptide in the test sample that is higher or lower than the level or amount of the polypeptide in the control sample, such that the difference is statistically significant, is indicative of an alteration in the expression of the polypeptide encoded by the *CD44, SPP1* or *HDC* nucleic acid, and is diagnostic for a susceptibility to type 2 diabetes. Alternatively, the composition of the polypeptide encoded by a *CD44, SPP1* or *HDC* nucleic acid in a test sample is compared with the composition of the polypeptide encoded by the *CD44, SPP1* or *HDC* nucleic acid in a control sample (e.g., the presence of different splicing variants). A difference in the composition of the polypeptide in the test sample, as compared with the composition of the polypeptide in the control sample, is diagnostic for a susceptibility to type 2 diabetes. In another aspect, both the level or amount and the composition of the polypeptide can be assessed in the test sample and in the control sample. A difference in the amount or level of the polypeptide in the test sample, compared to the control sample; a difference in composition in the test sample, compared to the control sample; or both a difference in the amount or level, and a difference in the composition, is indicative of a susceptibility to type 2 diabetes.

### Assessment for Markers and Haplotypes

Genomes of individuals exhibit sequence variability at many locations in the genome. In some instances, reference is made to alternative alleles at a polymorphic site without choosing a reference allele. Alternatively, a reference sequence can be referred to for a particular polymorphic site. The reference allele is sometimes referred to as the "wild-type" allele and it usually is chosen as either the first sequenced allele or as the allele from a "non-affected" individual (e.g., an individual that does not display a disease or abnormal phenotype). Alleles that differ from the reference are referred to as "variant" alleles.

Haplotypes are a combination of various genetic markers having particular alleles at polymorphic sites. The haplotype can comprise a combination of various genetic markers, therefore, detecting haplotypes can be accomplished by methods known in the art for detecting sequences at polymorphic sites. Certain methods of identifying relevant markers and SNPs include the use of linkage disequilibrium (LD) and/or LOD scores.

In certain methods described herein, an individual who is at-risk for type 2 diabetes is an individual in whom at least one at-risk marker or haplotype is identified. In one aspect, the at-risk marker or haplotype is one that confers a significant increased risk (or susceptibility) of type 2 diabetes. In one embodiment, significance associated with a marker or haplotype is measured by a relative risk. In a further embodiment, the significance is measured by a percentage. In one embodiment, a significant increased risk is measured as a relative risk of at least about 1.2, including but not limited to: 1.2, 1.3, 1.4, 1.5, 1.6, 1.7, 1.8 and 1.9. In a further embodiment, a relative risk of at least 1.2 is significant. In a further embodiment, a relative risk of at least about 1.5 is significant. In a further embodiment, a significant increase in risk is at least about 1.7 is significant. In a further embodiment, a significant increase in risk is at least about 20%, including but not limited to about 25%, 30%, 35%, 40%, 45%, 50%, 55%, 60%, 65%, 70%, 75%, 80%, 85%, 90%, 95% and 98%. In a further embodiment, a significant increase in risk is at least about 50%, and may be increased with a combination of at risk markers..

An at-risk marker or haplotype in, or comprising portions of, the *CD44, SPP1* or *HDC* gene, is one where the marker or haplotype is more frequently present in an individual at risk for type 2 diabetes (affected), compared to the frequency of its presence in a healthy individual (control), and wherein the presence of the marker or haplotype is indicative of susceptibility to type 2 diabetes. As an example of a simple test for correlation would be a Fisher-exact test on a two by two table. Given a cohort of chromosomes the two by two table is constructed out of the number of chromosomes that include both of the markers or haplotypes, one of the markers or haplotypes but not the other and neither of the markers or haplotypes.

An at-risk marker or haplotype described herein is an at-risk marker or haplotype within or near *CD44, SPP1* or *HDC* that significantly correlates with type 2 diabetes. In other aspects, an at-risk marker or haplotype comprises an at-risk marker or haplotype within or near *CD44, SPP1* or *HDC* that significantly correlates with susceptibility to type 2 diabetes. In particular embodiments, the marker or haplotype is associated with the LD blocks of *CD44, SPP1* or *HDC,* as described herein.

The frequencies of haplotypes in the patient and the control groups can be estimated using an expectation-maximization algorithm (Dempster et al., J. R. Stat. Soc. B, 39:1-38 (1977)). An implementation of this algorithm that can handle missing genotypes and uncertainty with the phase can be used. In a preferred embodiment, a p-value of <0.05 is indicative of a significant marker and/or haplotype association.

For single marker association to the disease, the Fisher exact test can be used to calculate two-sided p-values for each individual allele. All p-values are presented unadjusted for multiple comparisons unless specifically indicated. The presented frequencies (for microsatellites, SNPs and haplotypes) are allelic frequencies as opposed to carrier frequencies. To minimize any bias due the relatedness of the patients who were recruited as families for the linkage analysis, first and second-degree relatives can be eliminated from the patient list. Furthermore, the test can be repeated for association correcting for any remaining relatedness among the patients, by extending a variance adjustment procedure described in Risch, N. & Teng, J. (Genome Res., 8:1273-1288 (1998)), DNA pooling (ibid) for sibships so that it can be applied to general familial relationships, and present both adjusted and unadjusted p-values for comparison. The differences are in general very small as expected. To assess the significance of single-marker association corrected for multiple testing a randomization test using the same genotype data can be carried out. Cohorts of patients and controls can be randomized and the association analysis redone multiple times (e.g., up to 500,000 times) and the p-value is the fraction of replications that produced a p-value for some marker allele that is lower than or equal to the p-value we observed using the original patient and control cohorts.

For both single-marker and haplotype analyses, relative risk (RR) and the population attributable risk (PAR) can be calculated assuming a multiplicative model (haplotype relative risk model) (Terwilliger, J. D. & Ott, J., Hum. Hered. 42:337-46 (1992) and Falk, C. T. & Rubinstein, P, Ann. Hum. Genet. 51 (Pt 3):227-33 (1987)), i.e., the risks of the two alleles/haplotypes a person carries multiply. For example, if RR is the risk of A relative to a, then the risk of a person homozygote AA will be RR times that of a heterozygote Aa and RR² times that of a homozygote aa. The multiplicative model has a nice property that simplifies analysis and computations-haplotypes are independent, i.e., in Hardy-Weinberg equilibrium, within the affected population as well as within the control population. As a consequence, haplotype counts of the affected and controls each have multinomial distributions, but with different haplotype frequencies under the alternative hypothesis. Specifically, for two haplotypes, hᵢ and hⱼ, risk(hᵢ)/risk(hⱼ)=(fᵢ/pᵢ/(fⱼ/pⱼ), where f and p denote, respectively, frequencies in the affected population and in the control population. While there is some power loss if the true model is not multiplicative, the loss tends to be mild except for extreme cases. Most importantly, p-values are always valid since they are computed with respect to null hypothesis.

LD between pairs of markers can be calculated using the standard definition of D' and R² (Lewontin, R., Genetics 49:49-67 (1964); Hill, W. G. & Robertson, A. Theor. Appl. Genet. 22:226-231 (1968)). Using NEMO, frequencies of the two marker allele combinations are estimated by maximum likelihood and deviation from linkage equilibrium is evaluated by a likelihood ratio test. The definitions of D' and R² are extended to include microsatellites by averaging over the values for all possible allele combination of the two markers weighted by the marginal allele probabilities. When plotting all marker combinations to elucidate the LD structure in a particular region, we plot D' in the upper left corner and the p-value in the lower right corner. In the LD plots the markers can be plotted equidistant rather than according to their physical location, if desired.

In certain embodiments, marker and haplotype analysis involves defining a candidate susceptibility locus based on "haplotype blocks" (also called "LD blocks"). It has been reported that portions of the human genome can be broken into series of discrete haplotype blocks containing a few common haplotypes; for these blocks, linkage disequilibrium data provided little evidence indicating recombination (see, e.g., Wall., J. D. and Pritchard, J. K., Nature Reviews Genetics 4:587-597 (2003); Daly, M. et al., Nature Genet. 29:229-232 (2001); Gabriel, S. B. et al., Science 296:2225-2229 (2002); Patil, N. et al., Science 294:1719-1723 (2001); Dawson, E. et al., Nature 418:544-548 (2002); Phillips, M. S. et al., Nature Genet. 33:382-387 (2003)). There are two main methods for defining these haplotype blocks: blocks can be defined as regions of DNA that have limited haplotype diversity (see, e.g., Daly, M. et al., Nature Genet. 29:229-232 (2001); Patil, N. et al., Science 294:1719-1723 (2001); Dawson, E. et al., Nature 418:544-548 (2002); Zhang, K. et al., Proc. Natl. Acad. Sci. USA 99:7335-7339 (2002)), or as regions between transition zones having extensive historical recombination, identified using linkage disequilibrium (see, e.g., Gabriel, S. B. et al., Science 296:2225-2229 (2002); Phillips, M. S. et al., Nature Genet. 33:382-387 (2003); Wang, N. et al., Am. J. Hum. Genet. 71:1227-1234 (2002); Stumpf, M. P., and Goldstein, D. B., Curr. Biol. 13:1-8 (2003)). As used herein, the terms "haplotype block" or "LD block" includes blocks defined by either characteristic.

Representative methods for identification of haplotype blocks are set forth, for example, in U.S. Published Patent Application Nos. 20030099964, 20030170665, 20040023237 and 20040146870. Haplotype blocks can be used readily to map associations between phenotype and haplotype status. The main haplotypes can be identified in each haplotype block, and then a set of "tagging" SNPs or markers (the smallest set of SNPs or markers needed to distinguish among the haplotypes) can then be identified. These tagging SNPs or markers can then be used in assessment of samples from groups of individuals, in order to identify association between phenotype and haplotype. If desired, neighboring haplotype blocks can be assessed concurrently, as there may also exist linkage disequilibrium among the haplotype blocks.

In specific embodiments, a marker or haplotype associated with the LD blocks of *CD44, SPP1* or *HDC* is one in which the marker or haplotype is more frequently present in an individual at risk for type 2 diabetes (affected), compared to the frequency of its presence in a healthy individual (control), wherein the presence of the marker or haplotype is indicative of type 2 diabetes or a susceptibility to type 2 diabetes. In other embodiments, at-risk tagging markers in linkage disequilibrium with one or more markers associated with the LD blocks of *CD44, SPP1* or *HDC* are tagging markers that are more frequently present in an individual at risk for type 2 diabetes (affected), compared to the frequency of their presence in a healthy individual (control), wherein the presence of the tagging markers is indicative of increased susceptibility to type 2 diabetes. In a further embodiment, at-risk markers in linkage disequilibrium with one or more markers associated with the LD blocks of *CD44, SPP1* or *HDC* are markers that are more frequently present in an individual at risk for type II diabetes, compared to the frequency of their presence in a healthy individual (control), wherein the presence of the markers is indicative of susceptibility to type 2 diabetes.

### Screening methods

Also described herein are provides methods for identifying agents (e.g., fusion proteins, polypeptides, peptidomimetics, prodrugs, receptors, binding agents, antibodies, small molecules or other drugs, or ribozymes) which alter (e.g., increase or decrease) the activity of CD44 or a CD44 ligand, *e.g.* osteopontin, Hyaluronic acid, *etc.,* which otherwise interact with CD44. For example, in certain embodiments, such agents can be agents that bind to CD44; which have an inhibitory effect on, for example, activity of CD44; or which inhibit the ability of CD44 to interact with its cognate ligands.

Described herein are assays for screening candidate or test agents that bind to or modulate the activity of CD44 protein (or biologically active portion(s) thereof) or a CD44 ligand, as well as agents identifiable by the assays. Preferably candidate agents are further tested in a model for type 2 diabetes, e.g. any one of the animal models set forth in Chen and Wang (2005) Diabetes Obes Metab. 7(4):307-17, herein specifically referenced with respect to the disclosure of animal models useful in drug screening.

Test agents can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection.

In one embodiment, to identify agents which alter the activity of CD44, a cell, cell lysate, or solution containing or expressing CD44, or a fragment or derivative thereof, or a CD44 ligand, can be contacted with an agent to be tested; alternatively, the protein can be contacted directly with the agent to be tested. The level (amount) of activity is assessed either directly or indirectly, and is compared with the level of activity in a control, e.g. in the absence of the agent to be tested. If the level of the activity in the presence of the agent differs, by an amount that is statistically significant, from the level of the activity in the absence of the agent, then the agent is an agent that alters the activity.

Described herein is an assay for identifying agents that alter the expression of the CD44 gene or a ligand thereof (e.g., antisense nucleic acids, fusion proteins, polypeptides, peptidomimetics, prodrugs, receptors, binding agents, antibodies, small molecules or other drugs, or ribozymes), that alter expression (e.g., transcription or translation) of the gene. For example, a nucleic acid encoding a CD44 can be contacted with an agent to be tested. The solution can comprise, for example, cells containing the nucleic acid or cell lysate containing the nucleic acid; alternatively, the solution can be another solution that comprises elements necessary for transcription/translation of the nucleic acid. Cells not suspended in solution can also be employed, if desired. The level and/or pattern of CD44 expression is assessed, and is compared with the level and/or pattern of expression in a control. If the level and/or pattern in the presence of the agent differs, by an amount or in a manner that is statistically significant, from the level and/or pattern in the absence of the agent, then the agent is an agent that alters the expression.

In another embodiment described herein, agents that alter the expression of CD44 or a ligand thereof can be identified using a cell, cell lysate, or solution containing a nucleic acid encoding the promoter region of the gene or nucleic acid operably linked to a reporter gene. After contact with an agent to be tested, the level of expression of the reporter gene is assessed, and is compared with the level of expression in a control. If the level in the presence of the agent differs, by an amount or in a manner that is statistically significant, from the level in the absence of the agent, then the agent is an agent that alters the expression of CD44, as indicated by its ability to alter expression of a gene that is operably linked to the CD44 gene promoter.

Agents that alter the amounts of different splicing variants encoded by CD44 (e.g., an agent which enhances expression of a first splicing variant, and which inhibits expression of a second splicing variant), as well as agents which are agonists of activity of a first splicing variant and antagonists of activity of a second splicing variant, can easily be identified using these methods described above.

In other embodiments described herein, assays can be used to assess the impact of a test agent on the activity of a polypeptide in relation to a CD44 ligand. For example, a cell that expresses a CD44 ligand is contacted with CD44 in the presence of a test agent, and the ability of the test agent to alter the interaction between the CD44 and the CD44 binding agent is determined. Alternatively, a cell lysate or a solution containing the CD44 ligand can be used. Determining the ability of the test agent to bind to CD44 or a CD44 ligand can be accomplished, for example, by coupling the test agent with a radioisotope or enzymatic label such that binding of the test agent to the polypeptide can be determined by detecting the labeled with ¹²⁵I, ³⁵S, ¹⁴C or ³H, either directly or indirectly, and the radioisotope detected by direct counting of radioemmission or by scintillation counting.

Alternatively, test agents can be enzymatically labeled with, for example, horseradish peroxidase, alkaline phosphatase, or luciferase, and the enzymatic label detected by diagnosis of conversion of an appropriate substrate to product. It is also within the scope of this disclosure to determine the ability of a test agent to interact with the polypeptide without the labeling of any of the interactants. For example, a microphysiometer can be used to detect the interaction of a test agent with CD44 or a CD44 ligand without the labeling of either the test agent, CD44, or the CD44 ligand. McConnell, H. M. et al., Science 257:1906-1912 (1992). As used herein, a "microphysiometer" is an analytical instrument that measures the rate at which a cell acidifies its environment using a light-addressable potentiometric sensor (LAPS). Changes in this acidification rate can be used as an indicator of the interaction between ligand and polypeptide.

In more than one embodiment of the above assay methods described herein, it may be desirable to immobilize either the CD44 gene, the CD44 protein, the CD44 ligand, or other components of the assay on a solid support, in order to facilitate separation of complexed from uncomplexed forms of one or both of the proteins, as well as to accommodate automation of the assay. Binding of a test agent to the protein, or interaction of the protein with a binding agent in the presence and absence of a test agent, can be accomplished in any vessel suitable for containing the reactants. Examples of such vessels include microtiter plates, test tubes, and micro-centrifuge tubes. In one embodiment, a fusion protein (e.g., a glutathione-S-transferase fusion protein) can be provided which adds a domain that allows CD44, CD44 protein, or a CD44 ligand to be bound to a matrix or other solid support.

This disclosure further pertains to novel agents identified by the above-described screening assays. Accordingly, it is within the scope of this disclosure to further use an agent identified as described herein in the methods of treatment described herein. For example, an agent identified as described herein can be used to alter activity of a protein encoded by a CD44 gene, or to alter expression of CD44 by contacting the protein or the nucleic acid (or contacting a cell comprising the polypeptide or the nucleic acid) with the agent identified as described herein.

Kits (e.g., reagent kits) useful in the methods of diagnosis comprise components useful in any of the methods described herein, including for example, hybridization probes or primers as described herein (e.g., labeled probes or primers), reagents for detection of labeled molecules, restriction enzymes (e.g., for RFLP analysis), allele-specific oligonucleotides, antibodies which bind to altered or to non-altered (native) *CD44, SPP1* or *HDC* polypeptide, means for amplification of nucleic acids comprising a *CD44, SPP1* or *HDC* nucleic acid or for a portion of *CD44, SPP1* or *HDC,* or means for analyzing the nucleic acid sequence of a *CD44, SPP1* or *HDC* nucleic acid or for analyzing the amino acid sequence of a *CD44, SPP1* or *HDC* polypeptide as described herein, etc. In one aspect, the kit for diagnosing a susceptibility to type 2 diabetes can comprise primers for nucleic acid amplification of a region in the *CD44, SPP1* or *HDC* nucleic acid comprising the SNP sequence set forth in SEQ ID NO:1-SEQ ID NO:12 or an at-risk haplotype that is more frequently present in an individual having type 2 diabetes or who is susceptible to type 2 diabetes. The primers can be designed using portions of the nucleic acids flanking SNPs that are indicative of type 2 diabetes.

### EXPERIMENTAL

The following methods were used in the examples that are described further below to illustrate embodiments of the present invention.

Before the subject invention is further described, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an," and "the" include plural reference unless the context clearly dictates otherwise. Unless defined otherwise, all technical and scientific terms used herein have the same meaning as commonly understood to one of ordinary skill in the art to which this invention belongs.

The citation of any publication is for its disclosure prior to the filing date and should not be construed as an admission that the present invention is not entitled to antedate such publication by virtue of prior invention.

## Claims

1. An inhibitor of CD44, which inhibitor is an antibody, for use in a method of treating insulin resistance, the method comprising administering the inhibitor of CD44 to an individual having type 2 diabetes, or diagnosed as susceptible to type 2 diabetes.

2. An inhibitor of CD44 for use according to Claim 1, wherein the CD44 is expressed on adipose tissue macrophages or adipocytes.

3. The inhibitor of CD44 for use according to claim 1, wherein in the method the individual is diagnosed as susceptible to type 2 diabetes mellitus (T2D) prior to administering the antibody, wherein diagnosis of susceptibility comprises determining the presence or absence of a polymorphic allele in a biological sample comprising DNA or RNA from said individual, wherein the polymorphic allele comprises a single nucleotide polymorphism selected from the polymorphisms set forth in SEQ ID NO: 1-12, and wherein presence of the allele is indicative of a susceptibility to type 2 diabetes mellitus.

4. The inhibitor of CD44 for use according to Claim 3, wherein in the method the increased susceptibility is **characterized by** a relative risk (RR) or odds ratio (OR) of at least 1.2.

5. A kit for assessing susceptibility to type 2 diabetes mellitus in an individual, the kit comprising reagents for selectively determining the presence or absence of at least one polymorphic allele in a biological sample from said individual, wherein the polymorphic allele is selected from the polymorphisms set forth in SEQ ID NO:1-4, and wherein the presence of the at least one SNP indicates susceptibility to type 2 diabetes.

6. The kit according to Claim 5, comprising
(a) probes that specifically bind to at least one sequence set forth in SEQ ID NO:1-4, and optionally further comprising an array of probes that selectively bind to polymorphic alleles associated with a susceptibility to type 2 diabetes, and/or
(b) reagents for selectively determining the presence or absence of additional polymorphic alleles associated with a predisposition to type 2 diabetes.

7. The inhibitor for use according to Claim 1, wherein in the method glucose homeostasis of the individual is improved.

## Patentansprüche

1. CD44-Inhibitor, wobei der Inhibitor ein Antikörper ist, zur Verwendung in einem Verfahren zur Behandlung einer Insulinresistenz, wobei das Verfahren das Verabreichen des CD44-Inhibitors an ein Individuum mit Diabetes Typ 2 oder bei dem diagnostiziert wurde, dass eine Empfänglichkeit für Diabetes Typ 2 besteht, umfasst.

2. CD44-Inhibitor zur Verwendung nach Anspruch 1, worin das CD44 auf Adipositas-Gewebemakrophagen oder Adipozyten exprimiert wird.

3. CD44-Inhibitor zur Verwendung nach Anspruch 1, worin das Individuum in dem Verfahren vor der Verabreichung des Antikörpers als empfänglich für Diabetes mellitus Typ 2 (T2D) diagnostiziert wird, worin die Diagnose der Empfänglichkeit das Bestimmen des Vorhandenseins oder des Fehlens eines polymorphen Allels in einer biologischen Probe umfassend DNA oder RNA von dem Individuum umfasst, worin das polymorphe Allel einen Einzelnucleotid-Polymorphismus umfasst, der aus den in Seq.-ID Nr. 1-12 dargelegten Polymorphismen ausgewählt ist, und worin das Vorhandensein des Allels eine Empfänglichkeit für Diabetes mellitus Typ 2 angibt.

4. CD44-Inhibitor zur Verwendung nach Anspruch 3, worin die erhöhte Empfänglichkeit in dem Verfahren durch ein relatives Risiko (RR) oder Wahrscheinlichkeitsverhältnis (OR) von zumindest 1,2 gekennzeichnet ist.

5. Set zum Bewerten der Empfänglichkeit für Diabetes mellitus Typ 2 in einem Individuum, wobei das Set Reagenzien zum selektiven Bestimmen des Vorhandenseins oder des Fehlens von zumindest einem polymorphen Allel in einer biologischen Probe des Individuums umfasst, worin das polymorphe Allel aus den in Seq.-ID Nr. 1 - 4 dargelegten Polymorphismen ausgewählt ist, und worin das Vorhandensein von zumindest einem SNP eine Empfänglichkeit für Diabetes Typ 2 angibt.

6. Set nach Anspruch 5, das Folgendes umfasst:
(a) Sonden, die spezifisch an zumindest eine in Seq.-ID Nr. 1-4 dargelegte Sequenz binden, und das gegebenenfalls weiters eine Anordnung von Sonden umfasst, die selektiv an polymorphe Allele binden, die mit einer Empfänglichkeit für Diabetes Typ 2 in Zusammenhang stehen, und/oder
(b) Reagenzien zum selektiven Bestimmen des Vorhandenseins oder des Fehlens von zusätzlichen polymorphen Allelen, die mit einer Veranlagung für Diabetes Typ 2 in Zusammenhang stehen.

7. Inhibitor zur Verwendung nach Anspruch 1, worin in dem Verfahren die Glucosehomöostase des Individuums verbessert wird.

## Revendications

1. Inhibiteur de CD44, lequel inhibiteur est un anticorps, destiné à être utilisé dans un procédé de traitement de l'insulinorésistance, le procédé comprenant l'administration de l'inhibiteur de CD44 à un individu souffrant de diabète de type 2, ou diagnostiqué comme étant sensible au diabète de type 2.

2. Inhibiteur de CD44 destiné à être utilisé selon la revendication 1, où le CD44 est exprimé sur des macrophages du tissu adipeux ou des adipocytes.

3. Inhibiteur de CD44 destiné à être utilisé selon la revendication 1 où, dans le procédé, l'individu est diagnostiqué comme étant sensible au diabète de type 2 (DT2) avant l'administration de l'anticorps, où le diagnostic d'une sensibilité comprend la détermination de la présence ou de l'absence d'un allèle polymorphique dans un échantillon biologique comprenant de l'ADN ou de l'ARN dudit individu, où l'allèle polymorphique comprend un polymorphisme de nucléotide simple sélectionné parmi les polymorphismes décrits dans SEQ ID NO: 1-12, et où la présence de l'allèle indique une sensibilité au diabète de type 2.

4. Inhibiteur de CD44 destiné à être utilisé selon la revendication 3 où, dans le procédé, la sensibilité accrue est **caractérisée par** un risque relatif (RR) ou un rapport des cotes (RC) d'au moins 1,2.

5. Kit pour évaluer la sensibilité au diabète de type 2 chez un individu, le kit comprenant des réactifs pour sélectivement déterminer la présence ou l'absence d'au moins un allèle polymorphique dans un échantillon biologique dudit individu, où l'allèle polymorphique est sélectionné parmi les polymorphismes décrits dans SEQ ID NO: 1-4, et où la présence du au moins un SNP indique une sensibilité au diabète de type 2.

6. Kit selon la revendication 5, comprenant
(a) des sondes qui se lient spécifiquement à au moins une séquence décrite dans SEQ ID NO: 1-4, et comprenant en outre facultativement un réseau de sondes qui se lient sélectivement à des allèles polymorphiques associés à une sensibilité au diabète de type 2, et/ou
(b) des réactifs pour sélectivement déterminer la présence ou l'absence d'allèles polymorphiques supplémentaires associés à une prédisposition au diabète de type 2.

7. Inhibiteur destiné à être utilisé selon la revendication 1 où, dans le procédé, l'homéostasie du glucose de l'individu est améliorée.
